(19) Europäisches Patentamt
European Patent Office
Office européen des brevets

(11) EP 0 643 780 B2

(12) NEW EUROPEAN PATENT SPECIFICATION

(45) Date of publication and mention of the opposition decision:
**01.09.1999 Bulletin 1999/35**

(45) Mention of the grant of the patent:
**18.09.1996 Bulletin 1996/38**

(21) Application number: **93900721.7**

(22) Date of filing: **30.11.1992**

(51) Int Cl.[6]: **C23G 5/02**, C23G 5/04

(86) International application number:
**PCT/US92/10291**

(87) International publication number:
**WO 93/11280 (10.06.1993 Gazette 1993/14)**

# (54) MULTIPLE SOLVENT CLEANING SYSTEM

REINIGUNGSSYSTEM MIT MEHRFACHLOESUNGSMITTEL

SYSTEME DE NETTOYAGE A BASE DE SOLVANTS MULTIPLES

(84) Designated Contracting States:
**BE DE DK ES FR GB IE IT NL SE**

(30) Priority: **02.12.1991 US 801199**

(43) Date of publication of application:
**22.03.1995 Bulletin 1995/12**

(73) Proprietor: **MINNESOTA MINING AND MANUFACTURING COMPANY**
**Saint Paul, Minnesota 55133-3427 (US)**

(72) Inventors:
- **NALEWAJEK, David**
  **West Seneca, NY 14224 (US)**
- **BASU, Rajat, Subhra**
  **Williamsville, NY 14221 (US)**
- **WILSON, David, Paul**
  **East Amherst, NY 14051 (US)**
- **VAN DER PUY, Michael**
  **Cheektowaga, NY 14227 (US)**
- **SWAN, Ellen, Louise**
  **Ransomville, NY 14131 (US)**
- **LOGSDON, Peter, Brian**
  **North Tonawanda, NY 14120 (US)**
- **ZYHOWSKI, Gary, John**
  **Lancaster, NY 14086 (US)**
- **INGHAM, Hepburn**
  **Des Plaines, IL 60016 (US)**
- **HARNISH, Daniel, Franklin**
  **Orchard Park, NY 14127 (US)**
- **RODGERS, Joel, Edward**
  **Pales Verdes Estates, CA 90274 (US)**

(74) Representative: **Brock, Peter William et al**
**Urquhart-Dykes & Lord**
**1 Richfield Place**
**Richfield Avenue**
**Reading RG1 8EQ Berkshire (GB)**

(56) References cited:

| | |
|---|---|
| EP-A- 0 350 316 | EP-A- 0 431 458 |
| EP-A- 0 450 855 | WO-A-91/09105 |
| WO-A-91/11269 | WO-A-91/13846 |
| WO-A-92/07113 | WO-A-92/22678 |
| WO-A-93/05002 | WO-A-93/08240 |
| FR-A- 2 198 795 | JP-A- 63 229 185 |
| US-A- 3 881 949 | US-A- 3 904 430 |
| US-A- 5 023 009 | US-A- 5 023 010 |
| US-A- 5 073 288 | US-A- 5 073 290 |
| US-A- 5 076 956 | US-A- 5 100 572 |
| US-A- 5 104 454 | |

- **Cleaning and Contamination of Electronics Components and Assemblies, Electrochemical Publications Ltd., 1986; B.N. Ellis, pages 171-195**

EP 0 643 780 B2

## Description

The present invention relates to a process of cleaning contaminated articles, and more particularly, to the defluxing or degreasing of parts in a non-aqueous cleaning system utilizing an organic solvent for cleaning the parts and a nonflammable hydrofluorocarbon solvent.

Solvent vapor phase degreasing and defluxing is a process of immersing a soiled substrate (e.g., a printed circuit board or a fabricated metal, glass, ceramic, plastic or elastomer part or composite) into a boiling, nonflammable liquid such as a chlorocarbon or chlorofluorocarbon fluid or admixture, followed by-rinsing the part in a second tank or cleaning zone by immersion or distillate spray with a clean solvent which is the same chlorocarbon or chlorofluorocarbon as used in the first cleaning zone. The parts are then dried by maintaining the cooled part in the condensing vapors until temperature has reached equilibrium.

Solvent cleaning of various types of parts generally occurs in batch, hoist-assisted batch, conveyor batch, or in-line type conveyor degreaser and defluxer equipment. Such in-line conveyor degreaser and defluxer equipment are disclosed in co-pending U.S. Patent application Serial No. 07/410,216, filed September 21, 1989, (entitled Cold Air Lock Vapor Seal"), now U.S. Patent 5,007,179, commonly assigned to the assignee of the present invention. Parts may also be cleaned in open top defluxing or degreasing equipment, such as that disclosed in U.S. Patent Application Serial No. 07/587,893, filed September 25,1990, also commonly assigned. In both types of equipment, the entrance and/or exit ends of the equipment are generally in open communication with both the ambient environment and the solvent within the equipment. In order to minimize the loss of solvent from the equipment by either convection or diffusion, a common practice in the art is to use water-cooled or refrigerant-cooled coils which create a condensed vapor blanket over a hot or ambient zone region in the degreaser/defluxer tank, such as disclosed in U.S. Patent 4,261,111 to Rand.

Therefore, in the foregoing solvent vapor phase degreasing process, it is generally known to use a single organic chlorocarbon or chlorofluorocarbon (CFC) fluid to perform the cleaning, rinsing, and drying steps. The use of CFC-113 and Freon type solvents have been, in the past, particularly popular. However, the vapor diffusion thereof into the environment has been implicated in recent scientific studies to be one of many possible contributing causes to the undesirable global depletion of stratospheric ozone, and the production and use of such chlorofluorocarbons is currently regulated and will be phased out in the U.S. by the end of this decade. In response to environmental concern, hydrochlorofluorocarbon (HCFC) based solvents have been developed in the last few years to provide more environmentally acceptable alternatives to CFC based vapor phase decreasing and defluxing processes. While these materials have been shown to be excellent CFC substitutes for a variety of cleaning applications, they are considered to be an interim replacement to CFCs since they still process a small, but finite, ozone depletion potential, although it is much lower than that of the CFCs they are replacing. Hence, these HCFC solvents are also proposed for global phase out in the near future. It is generally believed that organic solvents which do not contain chlorine, bromide, or iodine atoms will not contribute to stratospheric ozone depletion. However, organic chemicals which do not contain the above halogen atoms, such as hydrocarbons, alcohols, esters, ethers ketones, etc., usually contain undesirable flammability or reactivity properties. Per fluorinated hydrocarbons and hydrofluorocarbons possess many desirable solvent properties: zero ozone depletion potential; stable, non-reactive, high compatibility with plastics; good water displacement potential; generally non-toxic and inert, and ideally suited to vapor phase solvent cleaning equipment. However, perfluorocarbons have been found to be very poor solvents for many common organic and inorganic soils, e.g., fluxes. Hydrofluorocarbons offer improved but still limited cleaning ability over perfluorocarbon as the amount of fluorine content on the molecule diminishes, but low fluorine-content hydrofluorocarbons may start to exhibit undesirable flammability properties comparable to their hydrocarbon analogs.

Other types of cleaning processes such as aqueous cleaning exist. Aqueous cleaning generally involves the cleaning of a substrate or a part in an aqueous solution of detergents or surfactants, followed by multiple rinsing steps with purified water. The part is then dried by lengthy evaporation in air or by energy intensive thermal drying machines. This process is not always desirable due to the high energy cost for drying and the additional capital investment and 5 operating cost burden to provide aqueous waste water cleanup required by state and local authorities before sewering to ground water.

Another cleaning process, semi-aqueous cleaning, consists of cleaning a substrate in a hydrocarbon solvent based on, for example, terpenes, esters, or petroleum distillates having a high affinity for oils, waxes, and greases being cleaned from the parts, with or without the aid of a surfactant. The cleaned substrate is rinsed of the high boiling hydrocarbon solvent with multiple rinsing steps using purified water. The hydrocarbon solvent is phase separated back to the wash sump while the aqueous effluent must be processed before sewering to ground water. Consequently, high costs associated with drying energy and with processing waste effluent are evident, similar to the before mentioned aqueous cleaning process. A further drawback is that the hydrocarbon solvent usually possesses a flash point and this must be carefully handled or blanketed with a nonflammable compressed gas such as nitrogen to avoid explosion. Nitrogen gas is much more fugitive than the dense vapors of a fluorocarbon contained in a condensing zone. Furthermore, in a number of applications, while the substrate to be cleaned may be compatible with the hydrocarbon solvent, some plastics or metals may be incompatible with the aqueous rinse solvent, resulting in water absorption or rusting of the substrate.

EP-A-0350316 describes a method of cleaning and drying components by contact with a hydrogencontaining flammable, liquid organic solvent wherein the solvent surface is covered by a vapour layer rich in a highly fluorinated organic compound which transfers heat to the organic solvent, the component to be cleaned is contacted with the liquid organic solvent, removed therefrom, vapour-rinsed or dried in the highly fluorinated compound-rich vapour layer and then removed from the cleaning environment.

EP-A-0350316 also describes an apparatus for cleaning and drying components, comprising a highly fluorinated compound reservoir means for heating and evaporating highly fluorinated compound from said reservoir, means for conducting highly fluorinated compound vapour to a cleaning chamber to cover an organic liquid in said chamber means for conducting components to be cleaned in and out of said chamber, and means for condensing and re-cycling highly fluorinated compound and organic solvent to their respective reservoirs.

EP-A-0431458 describes a cleaning composition in which the active component is an aliphatic fluorohydrocarbon of the formula:

$$C_n F_m H_{2n+2-m}$$

wherein $4 \le n \le 6$ and $6 \le m \le 12$ of the substrate.

JP-A-63-229185 discloses a cleaning method which comprises; (a) immersing a material to be cleaned in a mixed solvent contained in the first open vessel, wherein the mixed solvent consists of a chlorofluoro-hydrocarbon with a boiling point of 20°-50° C, and an organic solvent with a boiling point of 50° C, or more higher than that of said chlorofluoro-hydrocarbon and compatible therewith, but not azeotropic therewith, and kept at a temperature within a range between the boiling point of the chlorofluoro-hydrocarbon and a temperature of at least 30° C, lower than the boiling point of the organic solvent, (b) then transferring the material into a boiling liquid essentially consisting of said chlorofluoro-hydrocarbon contained in the second open vessel, (c) and taking out the material from the boiling liquid, (d) wherein, constituting a saturated vapor zone of the chlorofluoro-hydrocarbon by the evaporated vapor from the boiling liquid essentially consisting of the chlorofluoro-hydrocarbon and the mixed solvent in an open container equipped with cooling apparatus in the upper part and holding the open vessels in the lower part, (e) and keeping a constant state of the ratio of the chlorofluoro-hydrocarbon and the organic solvent in the mixed solvent, with a equilibrium of the evaporation of the chlorofluoro-hydrocarbon from the mixed solvent and its absorption from the saturated vapor zone of the chlorofluoro-hydrocarbon, by keeping the temperature of the mixed solvent at a constant temperature.

It is accordingly one object of the present invention to provide a non-aqueous cleaning process for degreasing or defluxing parts in an environmentally safe manner.

Another object of the invention is to provide a non-aqueous cleaning process which does not use water for rinsing, and there does not exist a necessity for aqueous waste water cleanup, and whereby said non aqueous cleaning system can be used in cases where materials are incompatible with water.

Still a further object is to provide a non-aqueous cleaning process avoiding the need for drying by lengthy evaporation of rinsing fluid- in air or by energy intensive thermal drying methods.

Yet a further object is to provide a non-aqueous cleaning process utilizing an organic liquid cleaning agent for cleaning the parts and a rinsing agent having at least a slight solubility for the organic cleaner for rinsing the organic cleaner from the part and which rinsing agent is capable of drying from the part using small amounts of energy.

The present invention provides a non-aqueous cleaning process for removing residual soils or surface contamination from a part, by immersing the part in an organic cleaning fluid of sufficient solvency to remove the soils or contamination from the surface of the part, rinsing the cleaned part with a rinsing solvent to remove the cleaning fluid, and drying the part.

According to the invention:

(a) the part is immersed in the cleaning fluid in a cleaning compartment,

(b) the cleaned part is transferred from the cleaning compartment to a rinsing compartment, and

(c) a flammability-suppression blanket consisting essentially of a substantially pure hydrofluorocarbon vapor is provided over the cleaning and rinsing compartments.

According to one embodiment of the invention, the rinsing compartment contains a hydrofluorocarbon-based rinsing solvent having a miscibility for organic cleaning fluids in the boiling range of at least 25°C to 120°C such that

at least 2 mole % of the organic cleaning fluid is miscible with the hydrofluorocarbon fluid without obtaining phase separation, and said hydrofluorocarbon-based rinsing solvent having a lesser solvency for the soils or contamination from the surface of the part than the organic cleaning fluid, said rinsing solvent being based on a hydrofluorocarbon compound consisting of hydrogen, carbon and fluorine and optionally a functional group selected from the group consisting of oxygen, sulphur, nitrogen and phosphorus atom.

According to another embodiment of the invention, the rinsing compartment contains a hydrofluorocarbon-based rinsing solvent having a miscibility for organic cleaning fluids in the boiling range of at least 25°C to 120°C such that at least 2 mole % of the organic cleaning fluid is miscible with the hydrofluorocarbon fluid without obtaining phase separation, said rinsing solvent being based on a linear or branched hydrofluorocarbon compound consisting of hydrogen, 3 to 8 carbon atoms and fluorine, the cleaning fluid and the rinsing solvent are not both an azeotrope formed by $CHF_2CF_2CF_2CHF_2$ and methanol, ethanol, n-propanol or isopropanol, or an azeotrope formed by $CF_3CH_2CF_2CF_2CF_3$ or $CF_3CF_2CH_2CF_2CF_3$ and methanol, ethanol or isopropanol.

The rinsing agent is based on a hydrofluorocarbon and may contain fluorocarbon solvents which contain at least one fluorine atom attached to an organic backbone comprised of two or more carbon atoms, with optionally other atoms also attached to the backbone such as oxygen, sulfur, nitrogen, phosphorus, hydrogen, or other halogen atoms. The parts are then dried by holding under an inert vapor blanket of hydrofluorocarbon which lessens or mitigates the flammability of the organic cleaning fluid.

In one embodiment the cleaning can be done in a system where a solution of the hydrocarbon solvent and the fluorocarbon solvent is mixed together optionally with a surfactant as a solubilizing agent in a degreaser.

The organic cleaning solvent can be selected from linear or branched alkyl or alkanol monocarboxylic or dicarboxylic esters having at least one carbon atom in the ester moiety and such solvent most preferably having a flash point greater than 93°C (200°F), or less preferably having a flash point greater than 66°C (150°F). The organic fluid may also be selected from linear or cyclic hydrocarbons containing at least one olefinic bond endo or exo to the ring. The hydrocarbon cleaning agent may comprise pinene and/or camphene, or may comprise terpinene, limonene, terpinolene, terpineol, linaleol, and other related members of the terpene family.

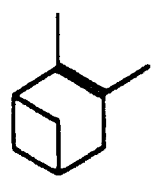

ALPHA-PINENE

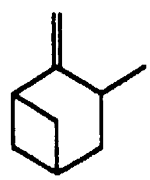

BETA-PINENE

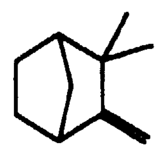

CAMPHENE

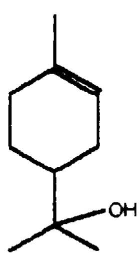

ALPHA-TERPINEOL

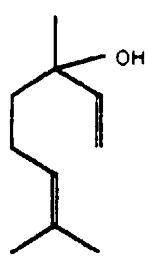

LINALOOL

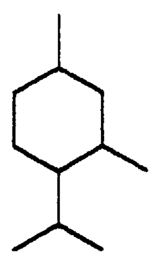

MENTHOL

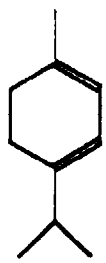

ALPHA-TERPINENE

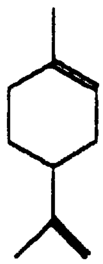

LIMONENE

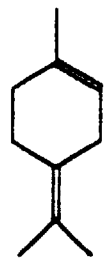

TERPINOLENE

The organic cleaning solvent may also consist of linear, branched or cyclic hydrocarbons containing $C_{10}$ to $C_{30}$ species.

The organic cleaning fluid may also consist of hydrocarbon containing olefinic moieties which have been substituted with $R_1$-$R_{12}$ groups, wherein $R_1$-$R_{12}$ hydrogen atoms or alkyl groups containing 1-6 carbon atoms or both may comprise the substituted group, i.e.,

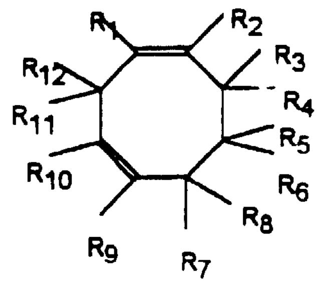

This organic cleaning fluid may also be comprised of acyclic or cyclic monols or diols defined by the linear structure (1)

$$R(CH_2)_nOH$$

$$R = H,\ \text{hydroxyl} \qquad (1)$$

where n is selected from 1 to 20 or the branched structure (2)

(2)

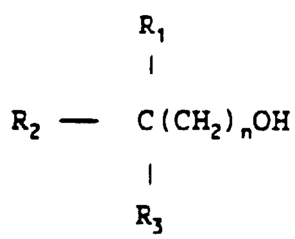

where n is selected from 1 to 20 or the cyclic structure (3)

(3)

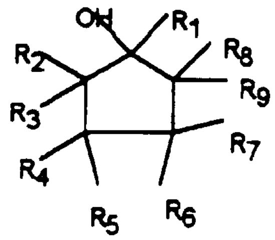

where $R_1$ - $R_9$ is defined as alkyl or hydrogen groups of mixtures thereof, and n is defined as 0 to 6.

The organic fluid may also be comprised of linear, branched, or cyclic mono or polyketones, such as

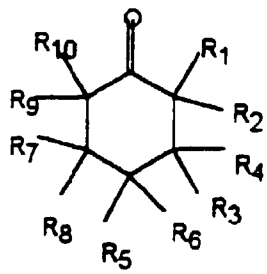

where n is defined as 0 to 6 and $R_1$-$R_{10}$ is defined as alkyl or hydrogen groups or mixtures thereof.

Other organic cleaning fluids applicable to this invention may be comprised of:

(a) alkyl or aryl nitriles of the formula:

$$R - CN$$

where R may be an alkyl group (methyl, ethyl, etc), a phenyl group, or an alkyl substituted phenyl group

(b) an alkylbenzene of the formula:

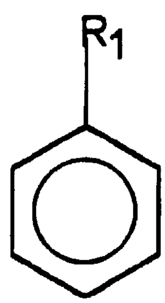

(c) alkyl esters such as diethylphthalate of the formula:

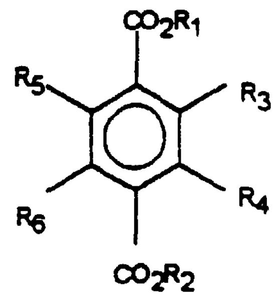

(d) polyether alkanols of the formula:

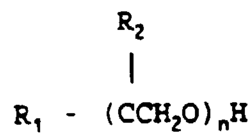

where $R_1$ is chosen from the class of compounds defined by H, alkyl, or hydroxyl groups and $R_2$ is selected from the class of compounds defined by H or alkyl or fluoroalkyl groups.

(e) substituted aromatics of the formula:

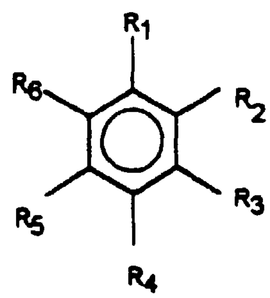

where $R_1$-$R_6$ is chosen from hydrogen, alkyl, fluoroalkyl, or halogen groups and combinations thereof, such as trifluorobenzene.

Such organic cleaning solvent may also comprise mixtures of all the above organic cleaning solvents with fluorocarbons such as linear, branched, or cyclic perfluorocarbons or hydrofluorocarbons or hydrochlorofluorocarbons optionally with substituted oxygen, sulfur, nitrogen, phosphorus or other halogen atoms attached to a carbon atoms and optionally with surfactant as a solubilizing agent.

The rinse solvent is selected from the class of hydrofluorocarbon compounds or mixtures thereof with linear, branched, or cyclic fluorocarbon linear, branched, or cyclic fluorocarbon structures having a boiling point of at least 25°C to 120°C and such fluorocarbons may be optionally substituted with other functional groups chosen from the class consisting of other halogens and oxygen, sulfur, nitrogen, and phosphorus atoms.

The hydrofluorocarbon compounds have a certain miscibility for organic cleaning solvents in the boiling range of at least 25°C to 120°C so that at least 2 mole % of the hydrocarbon cleaning solvent is miscible with the hydrofluorocarbon fluid without obtaining phase separation.

The hydrofluorocarbons preferably contain between 3 to 8 carbon atoms, hydrogen and fluorine in the compound The boiling point is preferably between 25°C and 120°C with at least 60% of fluorine in the compound. The compounds preferably have a linear or branched chain.

Providing flammability suppression can be maintained through the use of suitable vapor blanketing hydrofluorocarbon species.

Still other objects and advantages of the present invention will become readily apparent to those skilled in this art from the following detailed description, wherein only the preferred embodiments of the invention are shown and described, simply by way of illustration of the best mode contemplated of carrying out the invention. As will be realized, the invention is capable of other and different embodiments, and its several details are capable of modifications in

various obvious respects, all without departing from the invention. Accordingly, the drawings and description are to be regarded as illustrative in nature, and not as restrictive.

Brief Description of Drawings

Figure 1 is a partial schematic view of degreasing or defluxing equipment that may be used in the multi-solvent non-aqueous cleaning system of the prevent invention;

Figure 2 is a schematic view depicting an alternative embodiment of equipment that may be used in the present invention;

Figure 3 is a schematic view depicting a further alternative embodiment of equipment for use in the present invention;

Figure 4 is a partial schematic view of yet further alternative equipment for practicing the system of the prevent invention;

Figure 5 it another embodiment of equipment for use in the present inventions and;

Figure 6 is a further embodiment of equipment for use in the present invention.

In its most basic form, the present invention is a novel non-aqueous cleaning process whereby the most attractive features of the semi-aqueous cleaning process and the solvent vapor degreasing solvent process are combined. Specifically, a substrate or part to be cleaned (e.g., a printed circuit board coated with a rosin-based flux, or a metallic or nonmetallic part coated with a petroleum, synthetic, or semisynthetic based oil or grease) is first washed in a warm or ambient temperature hydrocarbon solvent tending to have a greater affinity for the soil or contaminant on the substrate than a fluorocarbon based solvent. The part is then rinsed by spray or immersion in a second tank or cleaning region containing a nonflammable hydrofluorocarbon solvent, preferably having a lower boiling point than the hydrocarbon solvent. The hydrofluorocarbon solvent has at least slight solubility for the hydrocarbon solvent and therefore rinses the hydrocarbon solvent from the surface of the part. The hydrofluorocarbon solvent is then dried from the part surface by evaporation in a known manner. The benefit of this process is that drying costs are minimized, waste water treatment costs and equipment and capital investments are virtually eliminated, and safety of operation is improved. Further, through the use of hydrofluorocarbon solvents in the rinse and drying steps, the environmental benefit is greatly improved over the chlorocarbon or chlorofluorocarbon solvent systems.

The organic cleaning agent is preferably a hydrocarbon or may be selected from linear or branched alkyl or alkanol monocarboxylic esters having at least six carbon atoms in the aliphatic moiety and at least one carbon atom in the ester moiety.

The organic hydrocarbon fluid may also be selected from linear or cyclic hydrocarbons containing at least one olefinic bond endo or exo to the ring.

The hydrocarbon may also be pinene and/or camphene.

The preferred hydrofluorocarbons are selected from the following groups:

(1) compounds with the empirical formula:

$$C_3H_nF_8\text{-}n$$

where $n \leq 4$

Representative examples of this class are:

$$CH_2FCH_2FCHF_2$$

$$CH_2FCF_2CFH_2$$

$$CF_2HCH_2CF_2H$$

$$CF_2HCFHCF_2H$$

(2) linear or branched compounds of the empirical formula:

$$C_4H_nF_{10-n}$$

where n ≤ 5

Representative examples of this class are:

$CHF_2(CF_2)_2CF_2H$ $CF_3CF_2CH_2CH_2F$

$CH_3CF(CHF_2)CHF_2$ $CF_3CH_2CF_2CH_2F$

$CH_3CHFCF_2CF_3$ $CF_3CH_2CH_2CF_3$

$CH_2FCF_2CF_2CH_2F_2$ $CHF_2CH(CF_3)CF_3$

$CHF(CF_3)CF_2CF_3$

(3) linear or branched compounds of the empirical formula:

$$C_5H_nF_{12-n}$$

where n ≤ 6

Representative examples of this class are:

$CH_3CHFCH_2CF_2CF_3$ $CF_3CH_2CF_2CH_2CF_3$

$CH_3CHFCHFCF_2CF_3$ $CF_3CH_2CH_2CF_2CF_3$

$CH_3CHFCF_2CF_2CF_3$ $CF_3CF_2CF_2CH_2CH_3$

$CH_3CF_2CF_2CF_2CF_3$ $CF_3CH_2CHFCH_2CF_3$

$CH_2FCF_2CF_2CF_2CF_3$ $CF_3CH_2CF_2CH_2CH_2F$

$CHF_2CF_2CF_2CF_2CF_3$

$CH_3CF(CF_2H)CHFCHF_2$

$CH_3CF(CHFCHF_2)CF_3$

$CH_3CH(CF_2CF_3)CF_3$

$CHF_2CH(CHF_2)CF_2CF_3$

$CHF_2CF(CHF_2)CF_2CF_3$

$CHF_2CF_2CF(CF_3)_2$

(4) linear or branched compounds of the empirical formula:

$$C_6H_nF_{14-n}$$

where $n \leq 7$

Representative examples of this class are:

$CHF_2(CF_2)_4CF_2H$ $CF_3CF_2CH_2CH_2CF_2CF_3$

$CH_2CH_2CH_2CF_2CF_2CF_3$ $(CF_3CH_2)_2CHCF_3$

$CH_3CH_2CFHCFHCF_2CF_3$

$CH_3CHFCF_2CHFCHFCF_3$

$CH_3FCHFCH_2CF_2CHFCF_3$

$CF_2HCHFCF_2CF_2CHFCF_2H$

$CH_2FCF_2CF_2CF_2CF_2CF_2H$

$CHF_2CF_2CF_2CF_2CF_2CHF_2$

$CHF_2CF_2CF_2CF_2CF_2CF_3$

$CH_3CH(CHFCH_2CF_3)CF_3$

$CH_3CF(CF_2H)CHFCHFCF_3$

$CH_3CF(CF_3)CHFCHFCF_3$

$CH_3CF_2CF(CF_3)CF_2CH_3$

$CH_3CF(CF_3)CF_2CF_2CF_3$

$CHF_2CF_2CH(CF_3)CF_2CF_3$

$CHF_2CF_2CF(CF_3)CF_2CF_3$

(5) linear or branched compounds of the empirical formula:

$$C_7H_nF_{16-n}$$

where n ≤ 8.

Representative examples of this class are:

$CH_3CH_2CH_2CHFCF_2CF_2CF_3$

$CH_3CHFCH_2CF_2CHFCF_2CF_3$

$CH_3(CF_2)_5CH_3$

$CH_3CH_2(CF_2)_4CF_3$

$CF_3CH_2CH_2(CF_2)_3CF_3$

$CH_2FCF_2CHF(CF_2)_3CF_3$

$CF_3CF_2CF_2CHFCHFCF_2CF_3$

$CF_3CF_3CF_2CHFCF_2CF_2CF_3$

$CH_3CH_2CH_2CHFCF(CF_3)_2$

$CH_3CH(CF_3)CF_2CF_2CF_2CH_3$

$CH_3CF(CF_3)CH_2)CFHCF_2CF_3$

$CH_3CF(CF_2CF_3CHFCF_2CF_3$

$CH_3CH_2CH(CF_3)CF_2CF_2CF_3$

$CHF_2CF(CF_3)(CF_2)_3CHF_2$

$CHF_2CF(CF_3)(CF_2)_3CF_3$

(6) Linear or branched compounds of the empirical formula $C_8H_nF_{18-n}$ where $n \leq 9$. Representative examples of this class are:

$CH_3CH_2CH_2CH_2CF_2CF_2CF_2CF_3$

$CH_3(CF_2)_6CH_3$

$CF_3(CF_2)_6CF_2H$

$CHF_2CF(CF_3)(CF_2)_4CF_3$

$CHF_2CF(CF_3)(CF_2)_4CHF_2$

$CH_3CH_2CH(CF_3)CF_2CF_2CF_2CF_3$

$CH_3CF(CF_2CF_3)CHFCF_2CF_2CF_3$

$CH_3CH_2CH_2CHFC(CF_3)_2CF_3$

$CH_3C(CF_3)_2CF_2CF_2CF_2CH_3$

$CH_3CH_2CH_2CF(CF_3)CF(CF_3)_2$

$CH_2FCF_2CF_2CHF(CF_2)_3CF_3$

Figure 1 is a partial schematic illustration of one type of apparatus which can be used in the present process. Therein, a vessel 10 is divided into three sumps: a cleaning sump 15, a wash sump 20, and a rinsing sump 25. The cleaning compartment 15 is separated by one or more walls 17, 19 from the second compartment 20, which contains a hydrofluorocarbon fluid 22 heated to its boiling point by a heater 34, to provide a nonflammable condensing vapor blanket 30 or a flammability suppression blanket over all the sumps 15, 20, 25 common to vessel 10. Compartment 20 also provides an area where the bulk of the soil and organic cleaning agent can be washed from the substrate by either immersing into the hydrofluorocarbon fluid contained in compartment 20 or by placement in a spray stream 18 of pure hydrofluorocarbon condensate, whereby the contaminated liquid drops into the sump below. The cleaning compartment 15 is adapted to contain a body of the organic cleaning solvent 24 tailored for the cleaning application, which could be an organic hydrocarbon as noted above and in the examples below for heavy duty cleaning, or a mixture of an organic hydrocarbon with a milder solvent such as a hydrofluorocarbon for less rigorous cleaning applications (where compatibility with the substrate may be of more important consideration). The washing compartment 20 is adapted to contain a rinsing agent 22 having at least slight solubility for the cleaning solvent 24. As used throughout this specification, "slight solubility" of the rinsing hydrofluorocarbon solvent for the hydrocarbon cleaning solvent is defined as $\geq 2$ mole % of the hydrocarbon is soluble in hydrofluorocarbon solvent. An appropriate rinsing agent 22 in the present invention is a hydrofluorocarbon based solvent as noted above and in the examples below Optionally, a

second rinsing compartment 25, which is at a cooler temperature than compartment 20, may be provided downstream from the first rinsing compartment, and is also adapted to contain the hydrofluorocarbon solvent rinsing agent. The purpose of compartment 25 is to provide a final immersion rinse for the substrate to remove trace residues of soil or cleaning solvent and also to cool the substrate so that the part is rinsed with pure condensing vapor in the vapor zone 30. The vapor zone 30 is formed above the respective compartments 15, 20, 25 and a cooling coil 32 of a type known in the art (such as disclosed in U.S. Patent 4,261,111 to Rand) defines the uppermost extent of the vapor zone 32 to condense vapor for return of condensate to compartment 25.

It is to be noted that the hydrocarbon cleaning solvent 24 and hydrofluorocarbon rinsing fluid 22 may be chosen for their respective mutual solubilities such that some means of physical separation such as phase separation may be employed to remove built-up soils as well as to recycle clean organic solvent back to its originating sump. Therefore, with reference to Figure 1, it is to be noted that a U-tube phase separator (not shown) or weir or skimmer may be used in conjunction with compartment 20 to separate or remove condensed hydrocarbon which will float on top of the hydrofluorocarbon, and this separation device (not shown) may be arranged so that the excess hydrocarbon fluid will flow back to the cleaning tank 15 from the rinsing tank 20.

The vessel 10 of Figure 1 is depicted as an open top type of defluxer or degreaser. However, it is to be understood that the vessel 10, in its schematic form, may also characterize an in-line type of degreaser or defluxer wherein conveyor means (not shown) may be used to successively convey the parts from the cleaning sump 15 to the rinsing sumps 20 and 25.

In Figure 2, the organic cleaning fluid in cleaning tank 15 may optionally be mixed with a hydrofluorocarbon type solvent. The cleaning fluid in this case would be warmed to a sufficient temperature to boil off the fluorocarbon, where the boiling point of the hydrofluorocarbon should be at least 10°C. lower than that of the organic hydrocarbon fluid. The mixture is heated with coils 33 so that the resulting vapor zone immediately overlying the hydrocarbon is essentially a hydrofluorocarbon-based nonflammable or flammability suppression vapor zone to minimize the possibility of explosion. The cleaning fluid mixture may or may not require a surfactant additive to ensure phase homogeneity between the organic hydrocarbon and the fluorocarbon solvents. The hydrofluorocarbon which was boiled off would be maintained at a constant concentration in compartment 15 by either returning the vapor condensate back to this compartment and/or pumping fluid from the rinse compartment(s) 20,25 back to this sump through control with a volume or level sensingtransducer (not shown). In this variation of Figure 1, the boiling rinse sump 20 may not be necessary, or it could function purely as a second rinse sump at any intermediate temperature between compartment 15 and compartment 25. In the three-sump option where sump 20 supplies the hydrofluorocarbon vapor to form the nonflammable blanket for vessel 10, sump 20 may be heated with heating coils 34 and heating coils 33 may not be necessary. In the two-sump option where sump 15 supplies the hydrofluorocarbon vapor to form the nonflammable blanket 30 for vessel 10, heating coil 33 would be preferred and heating coil 34 may not be necessary. In the two-sump option where sump 15 supplies the hydrofluorocarbon vapor to form the nonflammable blanket 30 for vessel 10, sump 20 may not be necessary and sump 25 would provide a cool liquid rinse immersion prior to bringing the cleaned substrate into the vapor condensing zone 30 for a final pure condensate rinse.

In Figure 3, the rinse sump 20 may contain a saturated solution of the hydrocarbon based cleaning fluid and the hydrofluorocarbon based rinsing solvent. The fluids are selected so that the hydrocarbon phase separates at some low concentration (i.e., less than 10 mole %) in the hydrofluorocarbon and floats to the top of the more dense hydrofluorocarbon, providing a cascading effect back into the cleaning sump 15. The rinse sump(s) 25a or 25b may also in turn cascade pure rinse solvent into sump 20 to maintain its level and also to directionally provide a flow skimming action to sweep the separated organic layer towards the boil sump 15.

In Figure 4, the boil sump 15 contains both hydrocarbon and hydrofluorocarbon based solvents of sufficient immiscibility to form layered cleaning zones 15a and 15b. The advantage of this arrangement is that the heating coil 33, which provides the nonflammable vapor blanket for vessel 10, is now immersed in a hydrofluorocarbon-rich phase which lessens the possibility of accidental fire if the liquid in the sump drops below its intended level. Furthermore, the boiling hydrofluorocarbon liquid now provides an agitation action the top, less dense organic hydrocarbon phase to assist in the cleaning performance. As in Figure 3, the hydrofluorocarbon rinse solvent and the hydrocarbon cleaning solvent can be cascaded or pumped back to their respective sumps to ensure that sump liquid volumes are maintained.

In Figure 5, the hydrocarbon cleaning zone 15 may be separated from the rinsing zones 20 and 25 by being provided with in separate structures 11 and 12. This configuration is intended to provide the ability to retrofit conventional solvent vapor degreasing or defluxing batch-type equipment such as represented by vessel 12 in Figure 5 with the cleaning process of this invention. The carry-over of organic solvent from vessel 11 to vessel 12 can be reduced by mechanical devices such as an air knife 37. Nitrogen or any other nonflammable compressed gas may be introduced into the vapor zone overlying the hydrocarbon sump 15 to reduce hydrocarbon flammability or the danger of explosion, as typical with many types of conventional semi-aqueous (organic cleaning/aqueous rinse) cleaning processes. In the hydrofluorocarbon boil sump 20, the residual hydrocarbon 20 carried over from cleaning sump 15 may be mechanically separated out as described in Figure 1 and recycled back with a pump 45 (as schematically depicted) in view of the

physical separation of the two cleaning zones which would prevent cascading as in the previous embodiments.

In Figure 6, it is presumed that the organic cleaning solvent in cleaning sump 15 is immiscible or of low miscibility with the hydrofluorocarbon-based rinsing solvent in rinsing sump 25. To prevent mixing of these solvents together and thereby potentially recontaminating the cleaned substrate, the first rinsing zone is provided with a coupling solvent (such as an alkanol like butanol, or another fluorochemical such as trifluorobenzene, or any other type of hydrocarbon) wherein the hydrofluorocarbon solvent is miscible with the coupling solvent. Preferably, the hydrofluorocarbon solvent has a lower boiling point than either the organic cleaning solvent or the coupling solvent. In this Case, the hydrofluorocarbon in sump 25 primarily serves the purpose of blanketing the flammable liquids in sumps 15 and 20 with a nonflammable vapor, and the level of liquid in rinse sump 20 which is comprised primarily of coupling solvent is maintained by makeup with hydrofluorocarbon liquid from sump 25. The substrate which has been rinsed in coupling solvent sump 20 is either subjected to a final immersion rinse in sumps(s) 25a and/or 25b or is held in the hydrofluorocarbon condensing vapor zone 42 for a final rinse, which can be suitably accomplished since the coupling-solvent is miscible with the hydrofluorocarbon solvent.

EXAMPLES

The following examples are used to demonstrate the unexpected cleaning performance observed when a soiled coupon is first immersed in an organic cleaning solvent followed by a rinse with a hydrofluorochemical solvent. In these studies, stainless steel coupons were coated with various commercial petroleum, semi-synthetic, and synthetic oils. The commercial petroleum oils are paraffinic, straight or branched chain saturated hydrocarbons. All of these oils are used in the metal working industry for cooling and lubricating purposes. The synthetic oils contain synthetic polymer with additives containing fatty acids and amines. The semisynthetic oils are mixtures of the petroleum and synthetic oils. The cleaning process used for tests to demonstrate this invention consisted of 30 second immersion of a coupon in the organic cleaing solvent followed by a 30 second immersion in the hydrofluorochemical solvent and a 30 second rinse above the liquid in the cooling coil zone with the condensing vapors of the fluorochemical solvent. The amount of soil on the coupon before and after cleaning was determined with a commercial $CO_2$ coulometer, which measures to microgram sensitivity the amount of organic residue, expressed in carbon units, on a surface. The sample of residue on the coupon is introduced into a combustion furnace via a sample boat, and is combusted in oxygen atmosphere at a temperature of 650°C. The resulting $CO_2$ and other combustion products pass through scrubbers to remove any interfering halogens, sulfur, nitrogen oxides and water. The gas then passes to the coulometer cell which contains an indicating solution. As the gas stream passes through the solution, $CO_2$ is quantitatively absorbed, and reacts with a chemical in the solution to produce a titratable acid. An electric current is then automatically adjusted to neutralize the solution, the total current is integrated, and the results displayed as micrograms of carbon. The sensitivity of this method is +/- 0.01 micrograms carbon, which is one of the most sensitive methods to reproducibly analyze carbon components on a surface. Since all oils cleaned in this invention are primarily organic in nature, the monitoring of carbon content is an excellent way to determine with high reproducibility and sensitivity the amount of organic soil on a substrate.

Example One

A ($C_9$ to $C_{11}$) methyl ester is used as the organic solvent. Spectroscopic characterization indicated a small quantity of a branched component. HFC 52-13 is a branched hydrofluorocarbon ($C_6F_{13}H$) used as the rinsing agent. The methyl ester is effective in removing petroleum based oil from metal coupons at room temperature, but a thin film of the methyl ester solvent remained after the cleaning process. The oil is not able to be cleaned from the surface of the coupon by HFC 52-13. However, the process of cleaning with the methyl ester, rinsing with HFC 52-13 followed by a rinse by the hydrofluorocarbon condensing vapors was highly effective in removing greater than 99.9% of the thin film of high boiling ester from the metal coupon without leaving a measurable trace of oil contaminant. Effectiveness of cleaning was assessed by weight measurements. In each of the examples below, blank coupons were determined to contain about 10 ug carbon on the surface.

Example Two

A dibasic ester cleaning solvent mixture was prepared in the laboratory by synthesizing the dimethyl esters of adipic acid, succinic acid, and glutaric acid in the proportions of 10 wt%22 wt%68 wt%, respectively. Soiled coupons were immersed in a mixture of the dibasic esters with HFC-365 ($CF_3CH_2CF_2CH_3$) at 56°C for 30 seconds, followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 11 |
| (b) | petroleum | no HFC rinse | 819 | 495 |
| (c) | petroleum | no ester rinse | 819 | 70 |
| (d) | synthetic | this invention | 508 | 10 |
| (e) | synthetic | no HFC rinse | 508 | 724 |
| (f) | synthetic | no ester rinse | 508 | 499 |

In the experiments with the petroleum and synthetic oils, using an organic cleaning step followed by a hydrofluorochemical vapor rinse step resulted in completely cleaning the coupon surface (>99.9% removal of carbon). However, in experiments (b) and (e), significant amount of carbon remained on the surface when the coupon was cleaned by a 30 second immersion in basic ester only followed by a 30 second drying in air without the HFC vapor rinse. In experiments (c) and (f), significant amount of carbon residue remained on the surface when the coupon was cleaned by a second immersion in HFC-365 followed by a 30 second drying in air without using the ester cleaning step. This example demonstrates that a hydrofluorochemical vapor rinse step is required to completely clean a soiled surface which has been immersed in either a dibasic ester or a mixture of a dibasic ester with hydrofluorochemical, and neither the ester alone nor the hydrofluorochemical solvent alone is sufficient to completely clean the surface.

Example Three

Soiled coupons were immersed in a 50/50 volume % mixture of cyclohexanone, a cyclic ketone, and HFC-365, a hydrofluorocarbon, for 30 seconds at 57-59°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 8.7 |
| (b) | petroleum | no HFC rinse | 819 | 1064 |
| (c) | petroleum | no ketone cleaning | 819 | 70 |
| (d) | synthetic | this invention | 508 | 3.7 |
| (e) | synthetic | no HFC rinse | 508 | 1475 |
| (f) | synthetic | no ketone cleaning | 508 | 499 |
| (g) | mineral oil | this invention | 950 | 7.5 |
| (h) | synthetic | this invention | 1033 | 14.9 |

In experiments (a) through (f) using an organic cleaning step followed by a hydrofluorochemical vapor rinse step resulted in completely cleaning the coupon surface (>99.9% removal of carbon). However, in experiments (b) and (e), a significant amount of carbon remained on the surface when the coupon was cleaned by a 30 second immersion in cyclohexanone only followed by a 30 second drying in air without the HFC vapor rinse. In experiments (c) and (f), a significant amount of carbon residue remained on the surface when the coupon was cleaned by a 30 second immersion in HFC-365 followed by a 30 second drying in air without using the ketone cleaning step. This example demonstrates that a hydrofluorochemical vapor rinse step is required to completely clean a soiled surface which has been immersed in either a ketone (cyclic or a cyclic) with hydrofluorochemical, and neither the ketone alone nor the hydrofluorochemical solvent alone is sufficient to completely clean the surface.

Example Four

Soiled coupons were immersed in a 50/50 volume % mixture of liquid cyclohexanol, a cyclic alkanol, and

HFC-365, a hydrofluorocarbon, for 30 seconds at 57-59°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 4 |
| (b) | petroleum | no HFC rinse | 819 | 2397 |
| (c) | petroleum | no alkanol cleaning | 819 | 70 |
| (d) | synthetic | this invention | 508 | 139.7 |
| (e) | synthetic | no HFC rinse | 508 | 1148 |
| (f) | synthetic | no alkanol cleaning | 508 | 499 |
| (g) | mineral oil | this invention | 950 | 12.7 |
| (h) | synthetic | this invention | 1033 | 10.2 |

In the experiments with petroleum oil, using an organic cleaning step followed by a hydrofluorochemical vapor rinse step resulted in completely cleaning the coupon surface (>99.9% removal of carbon). Since cyclohexanol is a fairly poor solvent in cleaning synthetic oil, as shown in experiments (b) and (e), most of the oil and the organic solvent film was able to be removed with the cleaning process of this invention as shown in experiment (d). Additionally, in experiments (b) and (e), a significant amount of carbon remained on the surface when the coupon was cleaned by a 30 second immersion in cyclohexanol only followed by a 30 second drying in air without the HFC vapor rinse. In experiments (c) and (f), significant amount of carbon residue remained on the surface when the coupon was cleaned by a 30 second immersion in HFC-365 followed by a 30 second drying in air without using the alkanol cleaning step. This example demonstrates that a hydrofluorochemical vapor rinse step is required to completely clean a soiled surface which has been immersed in an alkanol (cyclic or a cyclic), and neither the alkanol alone nor the hydrofluorochemical solvent alone is sufficient to completely clean the surface.

Example Five

Soiled coupons were immersed in a 50/50 volume % mixture of liquid 1, 5-dimethylcyclooctadiene, a cyclic olefin, and HFC-365, a hydrofluorocarbon, for 30 seconds at 57-59°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 10.1 |
| (b) | petroleum | no HFC rinse | 819 | 2953 |
| (c) | petroleum | no olefin cleaning | 819 | 70 |
| (d) | synthetic | this invention | 508 | 18.8 |
| (e) | synthetic | no HFC rinse | 508 | 2831 |
| (f) | synthetic | no olefin cleaning | 508 | 499 |
| (g) | mineral oil | this invention | 950 | 10 |
| (h) | synthetic | this invention | 1033 | 15.1 |

In the experiments with petroleum oil and synthetic oil, using an organic cleaning step followed by a hydrofluorochemical vapor rinse step resulted in completely cleaning the coupon surface (>99.8% removal of carbon). However, in experiments (b) and (e), significant amount of carbon remained on the surface when the coupon was cleaned by a 30 second immersion in 1,5-dimethylcyclooctadiene only followed by a 30 second drying in air without the HFC vapor rinse. In experiments (c) and (f), significant amount of carbon residue remained on the surface when the coupon was cleaned by a 30 second immersion in HFC-365 followed by a 30 second drying in air without using the olefin solvent cleaning

step. This example demonstrates that a hydrofluorochemical vapor rinse step is required to completely clean a soiled surface which has been immersed in an olefin (cyclic or acyclic), and neither the olefin alone nor the hydrofluorochemical solvent alone is sufficient to completely clean the surface.

Example Six

Soiled coupons were immersed in a 50/50 volume % mixture of liquid benzotrifluoride, a fluorinated aromatic chemical, and HFC-365, a hydrofluorocarbon, for 30 seconds at 57-59°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 13.3 |
| (b) | petroleum | no HFC rinse | 819 | 138 |
| (c) | petroleum | no fl. org. cleaning | 819 | 70 |
| (d) | synthetic | this invention | 508 | 16.1 |
| (e) | synthetic | no HFC rinse | 508 | 1022 |
| (f) | synthetic | no fl. org. cleaning | 508 | 499 |
| (g) | mineral oil | this invention | 950 | 13.7 |
| (h) | synthetic | this invention | 1033 | 127.9 |

In the experiments with petroleum oil and synthetic oil, using an organic cleaning step followed by a hydrofluorochemical vapor rinse step resulted in completely cleaning the coupon surface (>99.8% removal of carbon). However, in experiments (b) and (e), significant amount of carbon remained on the surface when the coupon was cleaned by a 30 second immersion in benzotrifluoride only followed by a second drying in air without the HFC vapor rinse. In experiments (c) and (f), significant amount of carbon residue remained on the surface when the coupon was cleaned by a 30 second immersion in HFC-365 followed by a 30 second drying in air without using the fluoroamatic cleaning step. This example demonstrates that a hydrofluorochemical vapor rinse step is required to completely clean a soiled surface which has been immersed in a fluorinated aromatic solvent, and neither the fluoroaromatic cleaning solvent alone nor the fluorochemical solvent alone is sufficient to completely clean the surface.

Example Seven

Soiled coupons were immersed in a 50 volume % mixture of polyethylene glycol (MW 200), a polyether diol, a methyl ester, and 50 volume % of HFC-365, a hydrofluorocarbon, for 30 seconds at 45-50°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 14 |
| (b) | petroleum | no HFC rinse | 819 | 1917 |
| (c) | petroleum | no PEG/ester cleaning | 819 | 70 |
| (d) | synthetic | this invention | 508 | 11 |
| (e) | synthetic | no HFC rinse | 508 | 1847 |
| (f) | synthetic | no PEG/ester cleaning | 508 | 499 |
| (g) | mineral oil | this invention | 950 | 12 |
| (h) | synthetic | this invention | 1033 | 13 |

In the experiments with petroleum oil and synthetic oil, using an organic cleaning step followed by a hydrofluorochemical vapor rinse step resulted in completely cleaning the coupon surface (>99.8% removal of carbon). However, in experiments (b) and (e), significant amount of carbon remained on the surface when the coupon was cleaned by a 30 second immersion in polyethylene glycol/methyl ester solvent mixture only followed by a 30 second drying in air without the HFC vapor rinse. In experiments (c) and (f), significant amount of carbon residue remained on the surface when the coupon was cleaned by a 30 second immersion in HFC-365 followed by a 30 second drying in air without using the glycol/ester solvent cleaning step. This example demonstrates that a hydrofluorochemical vapor rinse step is required to completely clean a soiled surface which has been immersed in a mixture of glycol with an ester, and neither the mixed organic solvent alone nor the hydrofluorochemical solvent alone is sufficient to completely clean the surface.

Example Eight

Soiled coupons were immersed in either a methyl ester, or a mixture of polyethylene glycol (MW 200), a polyether diol, and a methyl ester, for 30 seconds at 57-59°C followed by a 30 second immersion in HFC-52-13, a highly fluorinated alkane, at ambient temperature and a 30 second vapor rinse with the hydrofluorochemical solvent. The following results were noted:

The synthetic oil and the grease Valvoline were both removed from metal coupons to better than 99.9% using the PEG-200/methyl ester cleaning solvent mixture with HFC 52-13 as the rinse solvent in the process of this invention.

Example Nine

Soiled coupons were immersed in a 50/50 volume % mixture of BIOACT EC-7, a commercial blend of terpenes and non-ionic surfactants, and HFC-365, a hydrofluorocarbon, for 30 seconds at 57-59°C followed by a 30 second immersion in HFC-365 at ambient temperature and a 30 second vapor rinse with HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 15.3 |
| (b) | petroleum | no HFC rinse | 819 | 2221 |
| (c) | petroleum | no terpene cleaning | 819 | 36 |
| (d) | synthetic | this invention | 508 | 13.6 |
| (e) | synthetic | no HFC rinse | 508 | 2272 |
| (f) | synthetic | no terpene cleaning | 508 | 27.9 |
| (g) | mineral oil | this invention | 950 | 12 |
| (h) | mineral oil | no terpene cleaning | 950 | 94 |
| (i) | synthetic | this invention | 1033 | 13 |
| (j) | synthetic | no terpene cleaning | 1033 | 340 |

In the experiments with petroleum oil and synthetic oil, using an organic cleaning step followed by a hydrofluorochemical vapor rinse step resulted in virtally complete cleaning of the coupon surface (>99.6% removal of carbon). However, in experiments (b) and(e), significant amount of carbon remained on the surface when the coupon was cleaned by a 30 second immersion in the terpene solvent mixture only followed by a 30 second drying in air without the HFC vapor rinse. In experiments (c) and (f), significant amount of carbon residue remained on the surface when the coupon was cleaned by a 30 second immersion in HFC-365 followed by a 30 second drying in air without using the terpene solvent cleaning step. This example demonstrates that a hydrofluorochemical vapor rinse step is required to completely clean a soiled surface which has been immersed in a terpene Solvent, and neither the terpene solvent alone nor the hydrofluorochemical solvent alone is sufficient to completely clean the surface.

Example Ten

Soiled coupons were immersed in 50/50 volume % mixture of liquid cyclohexanone, a cyclic ketone, and HFC-365, a hydrofluorocarbon, for 30 seconds at 56-59°C followed by a 30 second immersion in a nonflammable constant-boiling blend of 5% HCFC-141b, a hydrochlorofluorocarbon, and 90% HFC-365, a hydrofluorocarbon, at ambient temperature and a 30 second vapor rinse with the condensing azeotropic vapors of HCFC141b/HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 18 |
| (b) | petroleum | no HFC/HCFC blend rinse | 819 | 2221 |
| (c) | petroleum | no ketone cleaning | 819 | 70 |
| (d) | synthetic | this invention | 508 | 29 |
| (e) | synthetic | no HFC/HCFC blend rinse | 508 | 2272 |
| (f) | synthetic | no keton cleaning | 508 | 499 |
| (g) | mineral oil | this invention | 950 | 15 |
| (h) | synthetic | this invention | 1033 | 25 |

In the experiments with petroleum oil and synthetic oil, using an organic cleaning step followed by a fluorochemical blend of HCFC and HFC vapor rinse step resulted in virtually complete cleaning of the coupon surface (>99.8% removal of carbon). However, in experiments (b) and (e), significant amount of carbon remained on the surface when the coupon was cleaned by a 30 second immersion in the ketone cleaning solvent only followed by a 30 second drying in air without the HCFC/HFC vapor rinse. In experiments (c), (f), (h) and (j), more carbon residue remained on the surface when the coupon was cleaned by a 30 second immersion in HCFC-141b/HFC-365 azeotrope followed by a 30 second drying in air than in the dual solvent process of this invention using the terpene solvent cleaning step. This example demonstrates that even when a strong fluorochemical rinse solvent such as a blend containing HFCF-141b is used, the combined organic (ketone) cleaning step followed by a fluorochemical rinse step produces greater cleaning than if a ketone solvent alone or a fluorochemical solvent alone is used to clean the substrate.

Example Eleven

Soiled coupons were immersed in a 50/50 volume % mixture of liquid cyclohexanone, a cyclic ketone, and HFC-365/FC-72 (95:5 by weight) for 30 seconds at 56-59°C followed by a 30 second immersion in a non segregating blend of 5% FC-72, a perfluorocarbon, and 95% HFC-365, a hydrofluorocarbon, at ambient temperature and a 30 second vapor rinse with the condensing azeotropic vapors of FC-72/HFC-365. the following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 22 |
| (b) | petroleum | no ketone cleaning | 819 | 44.3 |
| (c) | synthetic | this invention | 508 | 20.1 |
| (d) | synthetic | no ketone cleaning | 508 | 453 |
| (e) | mineral oil | this invention | 950 | 21.3 |
| (f) | mineral oil | no ketone cleaning | 950 | 550 |
| (g) | synthetic | this invention | 1033 | 20.4 |
| (h) | synthetic | no ketone cleaning | 1033 | 426 |

In the above experiments, using an organic cleaning step followed by a fluorochemical vapor rinse step resulted in complete cleaning of the coupon surface (99.9% removal of carbon). More carbon residue remained on the surface when the coupon was cleaned by a 30 second immersion in the mixture of FC-72/HFC-365 followed by a 30 second drying in air without using the cyclohexanone solvent cleaning step. This example demonstrates that a hydrofluorochemical vapor rinse step is required to completely clean a soiled surface which has been immersed in a ketone solvent. and neither the ketone solvent alone nor the fluorochemical solvent alone is sufficient to completely clean the surface. In addition, it is well known that perfluorocarbons such as FC-72 are very poor solvents for oils.

Example Twelve

Soiled coupons were immersed in liquid cyclohexanol, a cyclic alcohol, which was not admixed with the HFC prior to cleaning (as in the previous examples). The coupons were immersed for 30 seconds at 56-59°C followed by a 30 second immersion in HFC-365, a hydrofluorocarbon, at ambient temperature and a 30 second vapor rinse with the condensing azeotropic vapors of HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 18.5 |
| (b) | synthetic | this invention | 508 | 166 |
| (c) | mineral oil | this invention | 950 | 20.9 |
| (d) | synthetic | this invention | 1033 | 22.4 |

In this experiment, the cleaning ability of the organic cleaner segregated from the HFC was demonstrated. The results are within experimental error to those of Example 4, where the admixed organic/solvent system was evaluated. Interestingly, in this example the HFC/organic mixture in the cleaning sump cleaned better than if the cleaning sump contained only organic solvent (cyclohexanol).

Example Thirteen

Soiled coupons were immersed in liquid cyclohexanone, a cyclic ketone, which was not admixed with the HFC prior to cleaning (as in the previous examples). The coupons were immersed for 30 seconds at 56-59°C followed by a 30 second immersion in HFC-365, a hydrofluorocarbon, at ambient temperature and a 30 second vapor rinse with the condensing azeotropic vapors of HFC-365. The following results were noted:

| Expt. | Oil on Substrate | Cleaning Method | Micrograms (ug) Carbon on the Surface | |
|---|---|---|---|---|
| | | | Before Cleaning | After Cleaning |
| (a) | petroleum | this invention | 819 | 11.3 |
| (b) | synthetic | this invention | 508 | 12.2 |
| (c) | mineral oil | this invention | 950 | 11.7 |
| (d) | synthetic | this invention | 1033 | 10.3 |

In this experiment, the cleaning ability of the organic cleaner segregated from the HFC was demonstrated. The results are within experimental error to those of Example 12, where the admixed organic/solvent system was evaluated. It will be readily seen by one of ordinary skill in the art that the present invention fulfills all of the objects set forth above. After reading the foregoing specification, one of ordinary skill will be able to effect various changes, substitutions of equivalents and various other aspects of the invention as broadly disclosed herein. It is therefore intended that the protection granted hereon be limited only by the definition contained in the appended claims.

## Claims

1. A non-aqueous cleaning process for removing residual soils or surface contamination from a part, by immersing the part in an organic cleaning fluid of sufficient solvency to remove the soils or contamination from the surface of the part, rinsing the cleaned part with a rinsing solvent to remove the cleaning fluid, and drying the part, characterized in that

(a) the part is immersed in the cleaning fluid in a cleaning compartment (15)
(b) the cleaned part is transferred from the cleaning compartment (15) to a nnsing compartment (20, 25) containing a hydrofluorocarbon-based rinsing solvent having a miscibility for organic cleaning fluids in the boiling range of at least 25°C to 120°C such that at least 2 mole % of the organic cleaning fluid is miscible with the hydrofluorocarbon fluid without obtaining phase separation, and said hydrofluorocarbon-based rinsing solvent having a lesser solvency for the soils or contamination from the surface of the part than the organic cleaning fluid, said rinsing solvent being based on a hydrofluorocarbon compound consisting of hydrogen, carbon and fluorine and a functional group selected from the group consisting of oxygen, sulphur, nitrogen and phosphorus atom, and
(c) a flammability-suppression blanket (30) consisting essentially of a substantially pure hydrofluorocarbon vapor is provided over the cleaning and rinsing compartments.

**2.** A process according to claim 1, characterized in that the cleaning fluid comprises a mixture of:

(a) an organic component and
(b) a linear or branched hydrofluorocarbon component structure having 3 to 8 carbon atoms and at least 60 weight percent fluorine, wherein said organic component is present in an amount of at least 2 mole percent based on the total of said composition and capable of substantially removing said residual soils or surface contamination from said part.

**3.** A process according to claim 1, characterized in that the rinsing agent consists essentially of one or more linear or branched hydrofluorocarbon compounds having 3 to 8 carbon atoms and at least 60% by weight of fluorine, and having a boiling point of from 25 to 125°C.

**4.** A process according to any one of claims 1 to 3, characterized in that the cleaning compartment (15) contains an organic cleaning solvent mixed with a hydrofluorocarbon solvent, wherein the mixture is heated to a sufficient temperature to boil off at least some of the hydrofluorocarbon having a boiling point less than the hydrocarbon fluid whereby to form the flammability-suppression blanket (30).

**5.** A process according to any one of claims 1 to 4, characterized in that said rinsing solvent is based on a hydrofluorocarbon selected from :

(1) compounds with the empirical formula:

$$C_3H_nF_{8-n}$$

where $1 \leq n \leq 4$
(2) linear or branched compounds of the empirical formula:

$$C_4H_nF_{10-n}$$

where $1 \leq n \leq 5$
(3) linear or branched compounds of the empirical formula:

$$C_5H_nF_{12-n}$$

where $1 \leq n \leq 6$
(4) linear or branched compounds of the empirical formula:

$$C_6H_nF_{14-n}$$

where $1 \leq n \leq 7$

(5) linear or branched compounds of the empirical formula:

$$C_7H_nF_{16-n}$$

where $1 \leq n \leq 8$

(6) linear or branched compounds of the empirical formula:

$$C_8H_nF_{18-n}$$

where $1 \leq n \leq 9$

**6.** A process according to any one of claims 1 to 5, characterized in that said hydrofluorocarbon rinsing agent and said organic cleaning fluid are selected so that any cleaning fluid present in the rinsing compartment (20) containing the hydrofluorocarbon rinsing agent separates at a predetermined low concentration from the hydrofluorocarbon and floats to the top of the rinsing compartment (20) to provide a cascading effect of cleaning fluid back into the cleaning compartment (15).

**7.** A process according to claim 6, characterized in that there is a second rinsing compartment (25) containing substantially entirely only a hydrofluorocarbon and providing a cascading effect of substantially pure hydrofluorocarbon rinse solvent into the first rinsing compartment (20) to maintain a predetermined level thereof and to directionally provide a flow skimming action to sweep the separated cleaning fluid back toward the cleaning compartment.

**8.** A process according to claim 1, characterized in that it is carried out in apparatus in which the cleaning compartment (15) is housed in a structure which is separate from a structure containing the rinsing compartment (20, 25).

**9.** A non-aqueous cleaning process for removing residual soils or surface contamination from a part, by immersing the part in an organic cleaning fluid of sufficient solvency to remove the soils or contamination from the surface of the part, rinsing the cleaned part with a rinsing solvent to remove the cleaning fluid, and drying the part, characterized in that

(a) the part is immersed in the cleaning fluid in a cleaning compartment (15)

(b) the cleaned part is transferred from the cleaning compartment (15) to a rinsing compartment (20, 25) containing a hydrofluorocarbon-based rinsing solvent having a miscibility for organic cleaning fluids in the boiling range of at least 25°C to 120°C such that at least 2 mole % of the organic cleaning fluid is miscible with the hydrofluorocarbon fluid without obtaining phase separation, said rinsing solvent being based on a linear or branched hydrofluorocarbon compound consisting of hydrogen, 3 to 8 carbon atoms and fluorine, and

(c) a flammability-suppression blanket (30) consisting essentially of a substantially pure hydrofluorocarbon vapor is provided over the cleaning and rinsing compartments

with the proviso that the cleaning fluid and the rinsing solvent are not both an azeotrope formed by $CHF_2CF_2CF_2CHF_2$ and methanol, ethanol, n-propanol or isopropanol, or an azeotrope formed by $CF_3CH_2CF_2CF_2CF_3$ or $CF_3CF_2CH_2CF_2CF_3$ and methanol, ethanol or isopropanol.

**10.** A process according to claim 9, characterized in that the cleaning fluid comprises a mixture of:

(a) an organic component and

(b) a linear or branched hydrofluorocarbon component structure having 3 to 8 carbon atoms and at least 60 weight percent fluorine, wherein said organic component is present in an amount of at least 2 mole percent based on the total of said composition and capable of substantially removing said residual soils or surface contamination from said part.

**11.** A process according to any one of claims 9 or 10, characterized in that the cleaning compartment (15) contains an organic cleaning solvent mixed with a hydrofluorocarbon solvent, wherein the mixture is heated to a sufficient temperature to boil off at least some of the hydrofluorocarbon having a boiling point less than the hydrocarbon fluid whereby to form the flammability-suppression blanket (30).

**12.** A process according to any one of claims 9 to 11, characterized in that said rinsing solvent is based on a hydrofluorocarbon selected from:

(1) compounds with the empirical formula:

$$C_3H_nF_{8-n}$$

where $1 \leq n \leq 4$

(2) linear or branched compounds of the empirical formula:

$$C_4H_nF_{10-n}$$

where $1 \leq n \leq 5$

(3) linear or branched compounds of the empirical formula:

$$C_5H_nF_{12-n}$$

where $1 \leq n \leq 6$

(4) linear or branched compounds of the empirical formula:

$$C_6H_nF_{14-n}$$

where $1 \leq n \leq 7$

(5) linear or branched compounds of the empirical formula:

$$C_7H_nF_{16-n}$$

where $1 \leq n \leq 8$

(6) linear or branched compounds of the empirical formula:

$$C_8H_nF_{18-n}$$

where $1 \leq n \leq 9$.

**13.** A process according to any one of claims 9 to 12, characterized in that said hydrofluorocarbon rinsing agent and said organic cleaning fluid are selected so that any cleaning fluid present in the rinsing compartment (20) containing the hydrofluorocarbon rinsing agent separates at a predetermined low concentration from the hydrofluorocarbon and floats to the top of the rinsing compartment (20) to provide a cascading effect of cleaning fluid back into the cleaning compartment (15).

**14.** A process according to claim 13, characterized in that there is a second rinsing compartment (25) containing substantially entirely only a hydrofluorocarbon and providing a cascading effect of substantially pure hydrofluorocarbon rinse solvent into the first rinsing compartment (20) to maintain a predetermined level thereof and to directionally provide a flow skimming action to sweep the separated cleaning fluid back toward the cleaning compartment.

**15.** A process according to claim 9, characterized in that it is carried out in apparatus in which the cleaning compartment (15) is housed in a structure which is separate from a structure containing the rinsing compartment (20, 25).

**Patentansprüche**

1. Nichtwäßriges Reinigungsverfahren zur Entfernung von Schmutzrückständen oder Oberflächenverunreinigungen von einem Werkstück durch Eintauchen des Werkstücks in eine organische Reinigungsflüssigkeit mit einem zur Entfernung der Verschmutzungen oder Verunreinigungen von der Werkstückoberfläche ausreichendem Lösungsvermögen, Spülen des gereinigten Werkstücks mit einem Spüllösungsmittel zur Entfernung der Reinigungsflüssigkeit und Trocknen des Werkstücks, dadurch gekennzeichnet, daß man

   (a) das Werkstück in einer Reinigungskammer (15) in die Reinigungsflüssigkeit eintaucht,
   (b) das gereinigte Werkstück aus der Reinigungskammer (15) in eine Spülkammer (20, 25) überführt, die ein Spüllösungsmittel auf Fluorkohlenwasserstoffbasis enthält, das eine solche Mischbarkeit mit organischen Reinigungsflüssigkeiten im Siedebereich von mindestens 25°C bis 120°C aufweist, daß die organische Reinigungsflüssigkeit mit der Fluorkohlenwasserstoff-Flüssigkeit zu mindestens 2 Mol-% ohne Auftreten von Phasentrennung mischbar ist, und das ein geringeres Lösungsvermögen für die Verschmutzungen oder Verunreinigungen von der Werkstückoberfläche als die organische Reinigungsflüssigkeit aufweist und das auf einer Fluorkohlenwasserstoffverbindung aus Wasserstoff, Kohlenstoff und Fluor und einer funktionellen Gruppe aus der Gruppe bestehend aus Sauerstoff-, Schwefel-, Stickstoff- und Phosphoratom basiert, und
   (c) über der Reinigungs- und der Spülkammer für eine im wesentlichen aus weitgehend reinem Fluorkohlenwasserstoffdampf bestehende Brandschutzatmosphäre (30) sorgt.

2. Verfahren nach Anspruch 1, dadurch gekennzeichnet, daß die Reinigungsflüssigkeit ein Gemisch aus:

   (a) einer organischen Komponente und
   (b) einer linearen oder verzweigten Fluorkohlenwasserstoffkomponente mit 3 bis 8 Kohlenstoffatomen und mindestens 60 Gewichtsprozent Fluor darstellt, worin die organische Komponente in einer Menge von mindestens 2 Molprozent, bezogen auf die gesamte Zusammensetzung, vorliegt und zur weitgehenden Entfernung von Schmutzrückständen oder Oberflächenverunreinigungen vom Werkstück befähigt ist.

3. Verfahren nach Anspruch 1, dadurch gekennzeichnet, daß das Spülmittel im wesentlichen aus einer oder mehreren, linearen oder verzweigten Fluorkohlenwasserstoffverbindungen mit 3 bis 8 Kohlenstoffatomen und mindestens 60 Gew.-% Fluor besteht und einen Siedepunkt von 25 bis 125°C aufweist.

4. Verfahren nach einem der Ansprüche 1 bis 3, dadurch gekennzeichnet, daß die Reinigungskammer (15) ein organisches Reinigungsmittel in Abmischung mit einem Fluorkohlenwasserstoff-Lösungsmittel enthält, wobei man das Gemisch auf eine so hohe Temperatur erwärmt, daß der einen niedrigeren Siedepunkt als die Kohlenwasserstoffflüssigkeit aufweisende Fluorkohlenwasserstoff zumindest teilweise abdampft, so daß sich die Brandschutzatmosphäre (30) bildet.

5. Verfahren nach einem der Ansprüche 1 bis 4, dadurch gekennzeichnet, daß das Spüllösungsmittel auf einem Fluorkohlenwasserstoff, ausgewählt aus:

   (1) Verbindungen mit der Summenformel:

$$C_3H_nF_{8-n},$$

   wobei $1 \leq n \leq 4$
   (2) linearen oder verzweigten Verbindungen mit der Summenformel:

$$C_4H_nF_{10-n},$$

   wobei $1 \leq n \leq 5$
   (3) linearen oder verzweigten Verbindungen mit der Summenformel:

$$C_5H_nF_{12-n},$$

wobei $1 \leq n \leq 6$
(4) linearen oder verzweigten Verbindungen mit der Summenformel:

$$C_6H_nF_{14-n},$$

wobei $1 \leq n \leq 7$
(5) linearen oder verzweigten Verbindungen mit der Summenformel:

$$C_7H_nF_{16-n},$$

wobei $1 \leq n \leq 8$
(6) linearen oder verzweigten Verbindungen mit der Summenformel:

$$C_8H_nF_{18-n},$$

wobei $1 \leq n \leq 9$

basiert.

**6.** Verfahren nach einem der Ansprüche 1 bis 5, dadurch gekennzeichnet, daß man das Fluorkohlenwasserstoff-Spülmittel und die organische Reinigungsflüssigkeit so wählt, daß sich jegliche in der das Fluorkohlenwasserstoff-Spülmittel enthaltenden Spülkammer (20) vorhandene Reinigungsflüssigkeit bei einer vorbestimmten niedrigen Konzentration vom Fluorkohlenwasserstoff trennt und zur Spitze der Spülkammer (20) aufsteigt, so daß die Reinigungsflüssigkeit kaskadenartig in die Reinigungskammer (15) zurückfließt.

**7.** Verfahren nach Anspruch 6, dadurch gekennzeichnet, daß eine zweite Spülkammer (25) vorhanden ist, die im wesentlichen ausschließlich einen Fluorkohlenwasserstoff enthält und aus der weitgehend reines Fluorkohlenwasserstoff-Spüllösungsmittel kaskadenartig so in die erste Spülkammer (20) fließt, daß diese auf einem vorbestimmten Flüssigkeitsniveau bleibt und die abgetrennte Reinigungsflüssigkeit infolge der gerichteten Oberflächenströmung wieder in die Reinigungskammer zurückgeschwemmt wird.

**8.** Verfahren nach Anspruch 1, dadurch gekennzeichnet, daß man es in einer Vorrichtung durchführt, in der die Reinigungskammer (15) in einem von einem die Spülkammer (20, 25) enthaltenden Gehäuse abgetrennten Gehäuse untergebracht ist.

**9.** Nichtwäßriges Reinigungsverfahren zur Entfernung von Schmutzrückständen oder Oberflächenverunreinigungen von einem Werkstück durch Eintauchen des Werkstücks in eine organische Reinigungsflüssigkeit mit einem zur Entfernung der Verschmutzungen oder Verunreinigungen von der Werkstückoberfläche ausreichendem Lösungsvermögen, Spülen des gereinigten Werkstücks mit einem Spüllösungsmittel zur Entfernung der Reinigungsflüssigkeit und Trocknen des Werkstücks, dadurch gekennzeichnet, daß man

(a) das Werkstück in einer Reinigungskammer (15) in die Reinigungsflüssigkeit eintaucht,
(b) das gereinigte Werkstück aus der Reinigungskammer (15) in eine Spülkammer (20, 25) überführt, die ein Spüllösungsmittel auf Fluorkohlenwasserstoffbasis enthält, das eine solche Mischbarkeit mit organischen Reinigungsflüssigkeiten im Siedebereich von mindestens 25°C bis 120°C aufweist, daß die organische Reinigungsflüssigkeit mit der Fluorkohlenwasserstoff-Flüssigkeit zu mindestens 2 Mol-% ohne Auftreten von Phasentrennung mischbar ist, das auf einer linearen oder verzweigten Fluorkohlenwasserstoffverbindung aus Wasserstoff, 3 bis 8 Kohlenstoffatomen und Fluor basiert, und
(c) über der Reinigungs- und der Spülkammer für eine im wesentlichen aus weitgehend reinem Fluorkohlenwasserstoffdampf bestehende Brandschutzatmosphäre (30) sorgt,

mit der Maßgabe, daß es sich nicht sowohl bei der Reinigungsflüssigkeit als auch bei dem Spüllösungsmittel um ein Azeotrop aus $CHF_2CF_2CF_2CHF_2$ und Methanol, Ethanol, n-Propanol oder Isopropanol oder ein Azeotrop aus $CF_3CH_2CF_2CF_2CF_3$ oder $CF_3CF_2CH_2CF_2CF_3$ und Methanol, Ethanol oder Isopropanol handelt.

**10.** Verfahren nach Anspruch 9, dadurch gekennzeichnet, daß die Reinigungsflüssigkeit ein Gemisch aus:

(a) einer organischen Komponente und
(b) einer linearen oder verzweigten Fluorkohlenwasserstoffkomponente mit 3 bis 8 Kohlenstoffatomen und mindestens 60 Gewichtsprozent Fluor darstellt, worin die organische Komponente in einer Menge von mindestens 2 Molprozent, bezogen auf die gesamte Zusammensetzung, vorliegt und zur weitgehenden Entfernung von Schmutzrückständen oder Oberflächenverunreinigungen vom Werkstück befähigt ist.

**11.** Verfahren nach Anspruch 9 oder 10, dadurch gekennzeichnet, daß die Reinigungskammer (15) ein organisches Reinigungsmittel in Abmischung mit einem Fluorkohlenwasserstoff-Lösungsmittel enthält, wobei man das Gemisch auf eine so hohe Temperatur erwärmt, daß der einen niedrigeren Siedepunkt als die Kohlenwasserstoffflüssigkeit aufweisende Fluorkohlenwasserstoff zumindest teilweise abdampft, so daß sich die Brandschutzatmosphäre (30) bildet.

**12.** Verfahren nach einem der Ansprüche 9 bis 11, dadurch gekennzeichnet, daß das Spüllösungsmittel auf einem Fluorkohlenwasserstoff, ausgewählt aus:

(1) Verbindungen mit der Summenformel:

$$C_3H_nF_{8-n},$$

wobei $1 \leq n \leq 4$
(2) linearen oder verzweigten Verbindungen mit der Summenformel:

$$C_4H_nF_{10-n},$$

wobei $1 \leq n \leq 5$
(3) linearen oder verzweigten Verbindungen mit der Summenformel:

$$C_5H_nF_{12-n},$$

wobei $1 \leq n \leq 6$
(4) linearen oder verzweigten Verbindungen mit der Summenformel:

$$C_6H_nF_{14-n},$$

wobei $1 \leq n \leq 7$
(5) linearen oder verzweigten Verbindungen mit der Summenformel:

$$C_7H_nF_{16-n},$$

wobei $1 \leq n \leq 8$
(6) linearen oder verzweigten Verbindungen mit der Summenformel:

$$C_8H_nF_{18-n},$$

wobei $1 \leq n \leq 9$

basiert.

**13.** Verfahren nach einem der Ansprüche 9 bis 12, dadurch gekennzeichnet, daß man das Fluorkohlenwasserstoff-Spülmittel und die organische Reinigungsflüssigkeit so wählt, daß sich jegliche in der das Fluorkohlenwasserstoff-Spülmittel enthaltenden Spülkammer (20) vorhandene Reinigungsflüssigkeit bei einer vorbestimmten niedrigen Konzentration vom Fluorkohlenwasserstoff trennt und zur Spitze der Spülkammer (20) aufsteigt, so daß die Reinigungsflüssigkeit kaskadenartig in die Reinigungskammer (15) zurückfließt.

**14.** Verfahren nach Anspruch 13, dadurch gekennzeichnet, daß eine zweite Spülkammer (25) vorhanden ist, die im wesentlichen ausschließlich einen Fluorkohlenwasserstoff enthält und aus der weitgehend reines Fluorkohlenwasserstoff-Spüllösungsmittel kaskadenartig so in die erste Spülkammer (20) fließt, daß diese auf einem vorbestimmten Flüssigkeitsniveau bleibt und die abgetrennte Reinigungsflüssigkeit infolge der gerichteten Oberflächenströmung wieder in die Reinigungskammer zurückgeschwemmt wird.

**15.** Verfahren nach Anspruch 9, dadurch gekennzeichnet, daß man es in einer Vorrichtung durchführt, in der die Reinigungskammer (15) in einem von einem die Spülkammer (20, 25) enthaltenden Gehäuse abgetrennten Gehäuse untergebracht ist.

**Revendications**

**1.** Procédé de nettoyage non aqueux pour éliminer les salissures résiduelles ou la contamination des surfaces d'une pièce, par immersion de la pièce dans un fluide nettoyant organique de pouvoir solvant suffisant pour éliminer les salissures ou la contamination de la surface de la pièce, rinçage de la pièce nettoyée avec un solvant de rinçage pour éliminer le fluide nettoyant, et séchage de la pièce, caractérisé en ce que

(a) la pièce est immergée dans le fluide nettoyant, dans un compartiment de nettoyage (15),
(b) la pièce nettoyée est transférée du compartiment de nettoyage (15) dans un compartiment de rinçage (20, 25) contenant un solvant de rinçage à base d'hydrocarbure fluoré ayant une miscibilité telle que, pour les fluides nettoyants organiques ayant un domaine d'ébullition d'au moins 25°C à 120°C, au moins 2% en moles du fluide nettoyant organique soit miscible avec le fluide d'hydrocarbure fluoré sans l'obtention d'une séparation des phases, et ledit solvant de rinçage à base d'hydrocarbure fluoré ayant un pouvoir solvant pour les salissures ou la contamination de la surface de la pièce moins grand que le fluide nettoyant organique, ledit solvant de rinçage étant à base d'un composé hydrocarbure fluoré constitué d'hydrogène, de carbone et de fluor et d'un groupe fonctionnel choisi dans le groupe constitué d'un atome d'oxygène, de soufre, d'azote et de phosphore, et
(c) une couverture anti-inflammabilité (30), essentiellement constituée d'une vapeur d'hydrocarbure fluoré pratiquement pure, est fournie sur les compartiments de nettoyage et de rinçage.

**2.** Procédé selon la revendication 1, caractérisé en ce que le fluide nettoyant comprend un mélange:

(a) d'un constituant organique et
(b) d'une structure de constituant hydrocarbure fluoré, linéaire ou ramifié, comportant de 3 à 8 atomes de carbone et au moins 60% en poids de fluor, dans lequel ledit constituant organique est présent à raison d'au moins 2% en moles, par rapport au total de ladite composition, et est capable d'éliminer substantiellement lesdites salissures résiduelles ou ladite contamination des surfaces de ladite pièce.

**3.** Procédé selon la revendication 1, caractérisé en ce que l'agent de rinçage est essentiellement constitué d'un ou de plusieurs composés hydrocarbures fluorés, linéaires ou ramifiés, comportant de 3 à 8 atomes de carbone et au moins 60% en poids de fluor, et possédant un point d'ébullition de 25 à 125°C.

**4.** Procédé selon l'une quelconque des revendications 1 à 3, caractérisé en ce que le compartiment de nettoyage (15) contient un solvant nettoyant organique mélangé avec un solvant hydrocarbure fluoré, dans lequel le mélange est chauffé à une température suffisante pour éliminer par ébullition au moins une partie de l'hydrocarbure fluoré possédant un point d'ébullition inférieur à celui du fluide hydrocarboné, afin de former la couverture anti-inflammabilité (30).

**5.** Procédé selon l'une quelconque des revendications 1 à 4, caractérisé en ce que ledit solvant de rinçage est à base d'un hydrocarbure fluoré choisi parmi:

(1) les composés de formule empirique:

$$C_3H_nF_{8-n'}$$

dans laquelle $1 \leq n \leq 4$

(2) les composés linéaires ou ramifiés de formule empirique:

$$C_4H_nF_{10-n'}$$

dans laquelle $1 \leq n \leq 5$

(3) les composés linéaires ou ramifiés de formule empirique:

$$C_5H_nF_{12-n'}$$

dans laquelle $1 \leq n \leq 6$

(4) les composés linéaires ou ramifiés de formule empirique:

$$C_6H_nF_{14-n'}$$

dans laquelle $1 \leq n \leq 7$

(5) les composés linéaires ou ramifiés de formule empirique:

$$C_7H_nF_{16-n'}$$

dans laquelle $1 \leq n \leq 8$

(6) les composés linéaires ou ramifiés de formule empirique:

$$C_8H_nF_{18-n'}$$

dans laquelle $1 \leq n \leq 9$

**6.** Procédé selon l'une quelconque des revendications 1 à 5, caractérisé en ce que ledit agent de rinçage hydrocarbure fluoré et ledit fluide nettoyant organique sont choisis de telle sorte que tout fluide nettoyant présent dans le compartiment de rinçage (20) contenant l'agent de rinçage hydrocarbure fluoré se sépare, à une faible concentration prédéterminée de l'hydrocarbure fluoré et flotte vers le haut du compartiment de rinçage (20) pour fournir un effet de cascade du fluide nettoyant retournant dans le compartiment de nettoyage (15).

**7.** Procédé selon la revendication 6, caractérisé en ce qu'il existe un second compartiment de rinçage (25) contenant pratiquement entièrement un hydrocarbure fluoré seulement et fournissant un effet de cascade du solvant de rinçage hydrocarbure fluoré pratiquement pur dans le premier compartiment de rinçage (20) afin de maintenir un niveau prédéterminé de ce solvant et de fournir, selon une certaine direction, une action d'écumage par écoulement pour balayer le fluide nettoyant séparé en le renvoyant vers le compartiment de nettoyage.

**8.** Procédé selon la revendication 1, caractérisé en ce qu'il est mis en oeuvre dans un appareil dans lequel le compartiment de nettoyage (15) est logé dans une structure séparée d'une structure contenant le compartiment de rinçage (20, 25).

**9.** Procédé de nettoyage non aqueux pour éliminer les salissures résiduelles ou la contamination des surfaces d'une pièce, par immersion de la pièce dans un fluide nettoyant organique de pouvoir solvant suffisant pour éliminer les salissures ou la contamination de la surface de la pièce, rinçage de la pièce nettoyée avec un solvant de rinçage pour éliminer le fluide nettoyant, et séchage de la pièce, caractérisé en ce que

(a) la pièce est immergée dans le fluide nettoyant, dans un compartiment de nettoyage (15),
(b) la pièce nettoyée est transférée du compartiment de nettoyage (15) dans un compartiment de rinçage (20,

25) contenant un solvant de rinçage à base d'hydrocarbure fluoré ayant une miscibilité telle que, pour les fluides nettoyants organiques ayant un domaine d'ébullition d'au moins 25°C à 120°C, au moins 2% en moles du fluide nettoyant organique soit miscible avec le fluide d'hydrocarbure fluoré sans l'obtention d'une séparation des phases, ledit solvant de rinçage étant à base d'un hydrocarbure fluoré linéaire ou ramifié constitué d'hydrogène, de 3 à 8 atomes de carbone et de fluor, et
(c) une couverture anti-inflammabilité (30), essentiellement constituée d'une vapeur d'hydrocarbure fluoré pratiquement pure, est fournie sur les compartiments de nettoyage et de rinçage;

à la condition que le fluide nettoyant et le solvant de rinçage ne soient pas tous deux un azéotrope formé par $CHF_2CF_2CF_2CHF_2$ et le méthanol, l'éthanol, le n-propanol ou l'isopropanol, ou un azéotrope formé par $CF_3CH_2CF_2CF_2CF_3$ ou $CF_3CF_2CH_2CF_2CF_3$ et le méthanol, l'éthanol ou l'isopropanol.

**10.** Procédé selon la revendication 9, caractérisé en ce que le fluide nettoyant comprend un mélange:

(a) d'un constituant organique et
(b) d'une structure de constituant hydrocarbure fluoré linéaire ou ramifié dont la structure comporte de 3 à 8 atomes de carbone et au moins 60 pour cent en poids de fluor, dans lequel ledit constituant organique est présent à raison d'au moins 2% en moles, par rapport au total de ladite composition, et est capable d'éliminer substantiellement lesdites salissures résiduelles ou ladite contamination des surfaces de ladite pièce.

**11.** Procédé selon l'une quelconque des revendications 9 ou 10, caractérisé en ce que le compartiment de nettoyage (15) contient un solvant nettoyant organique mélangé avec un solvant hydrocarbure fluoré, dans lequel le mélange est chauffé à une température suffisante pour éliminer par ébullition au moins une partie de l'hydrocarbure fluoré possédant un point d'ébullition inférieur à celui du fluide hydrocarboné, afin de former la couverture anti-inflammabilité (30).

**12.** Procédé selon l'une quelconque des revendications 9 à 11, caractérisé en ce que ledit solvant de rinçage est à base d'un hydrocarbure fluoré choisi parmi:

(1) les composés de formule empirique:

$$C_3H_nF_{8-n'}$$

dans laquelle $1 \leq n \leq 4$
(2) les composés linéaires ou ramifiés de formule empirique:

$$C_4H_nF_{10-n'}$$

dans laquelle $1 \leq n \leq 5$
(3) les composés linéaires ou ramifiés de formule empirique:

$$C_5H_nF_{12-n'}$$

dans laquelle $1 \leq n \leq 6$
(4) les composés linéaires ou ramifiés de formule empirique:

$$C_6H_nF_{14-n'}$$

dans laquelle $1 \leq n \leq 7$
(5) les composés linéaires ou ramifiés de formule empirique:

$$C_7H_nF_{16-n'}$$

dans laquelle $1 \leq n \leq 8$

(6) les composés linéaires ou ramifiés de formule empirique:

$$C_8H_nF_{18-n'}$$

dans laquelle $1 \leq n \leq 9$

**13.** Procédé selon l'une quelconque des revendications 9 à 12, caractérisé en ce que ledit agent de rinçage hydrocarbure fluoré et ledit fluide nettoyant organique sont choisis de telle sorte que tout fluide nettoyant présent dans le compartiment de rinçage (20) contenant l'agent de rinçage hydrocarbure fluoré se sépare, à une faible concentration prédéterminée de l'hydrocarbure fluoré, et flotte vers le haut du compartiment de rinçage (20) pour fournir un effet de cascade du fluide nettoyant retournant dans le compartiment de nettoyage (15).

**14.** Procédé selon la revendication 13, caractérisé en ce qu'il existe un second compartiment de rinçage (25) contenant pratiquement entièrement un hydrocarbure fluoré seulement et fournissant un effet de cascade du solvant de rinçage hydrocarbure fluoré pratiquement pur dans le premier compartiment de rinçage (20) afin de maintenir un niveau prédéterminé de ce solvant et de fournir, selon une certaine direction, une action d'écumage par écoulement pour balayer le fluide nettoyant séparé en le renvoyant vers le compartiment de nettoyage.

**15.** Procédé selon la revendication 9, caractérisé en ce qu'il est mis en oeuvre dans un appareil dans lequel le compartiment de nettoyage (15) est logé dans une structure séparée d'une structure contenant le compartiment de rinçage (20, 25).

FIG. 1

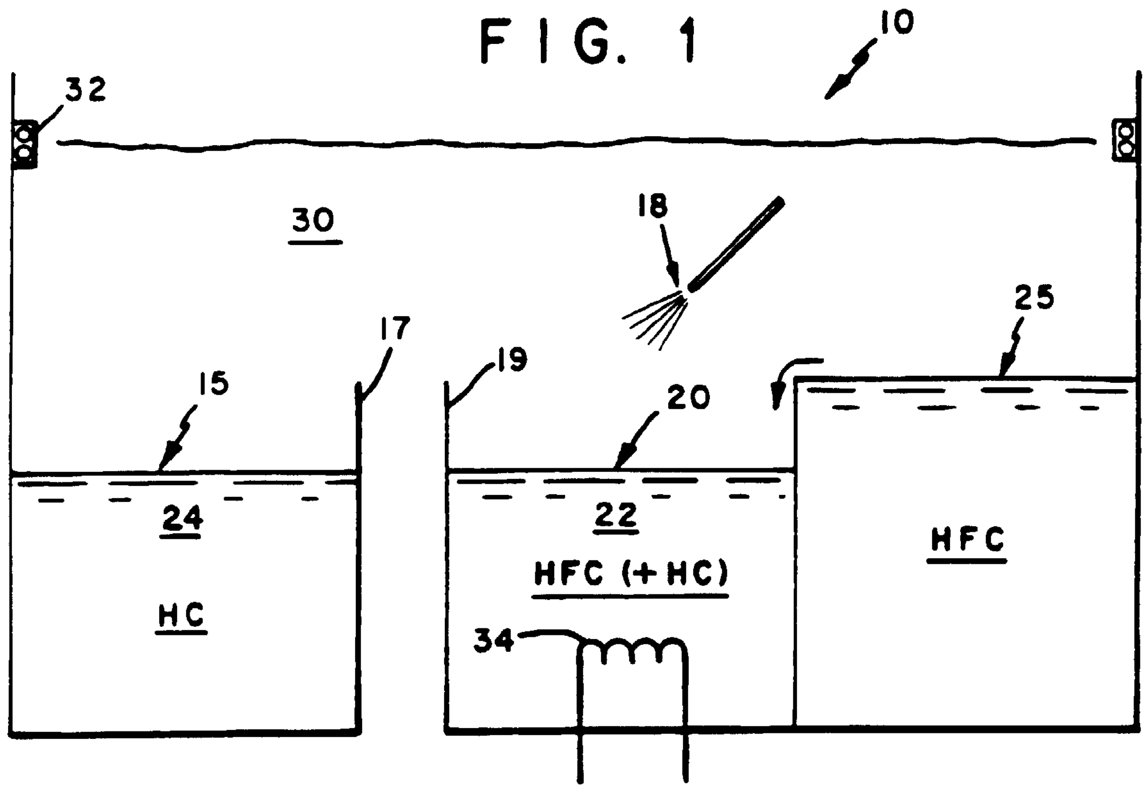
10
32
30
18
25
17
19
15
20
24
22
HFC (+HC)
HFC
HC
34

FIG. 2

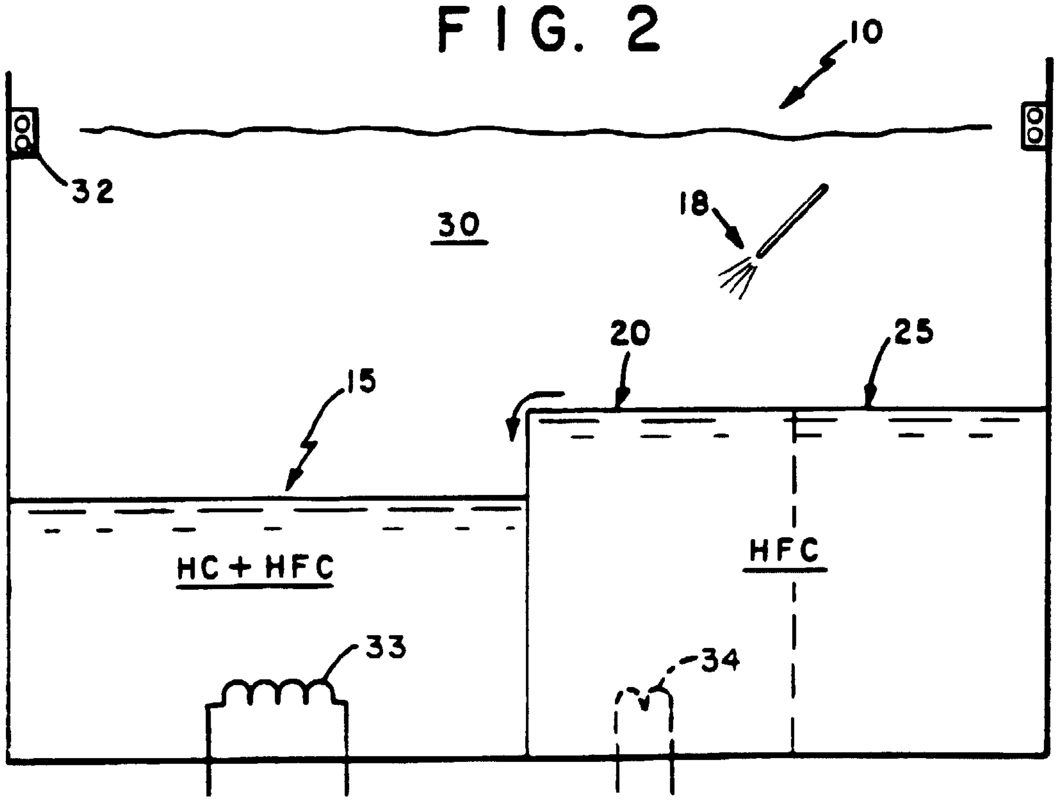
10
32
30
18
20
25
15
HC + HFC
HFC
33
34

FIG. 3

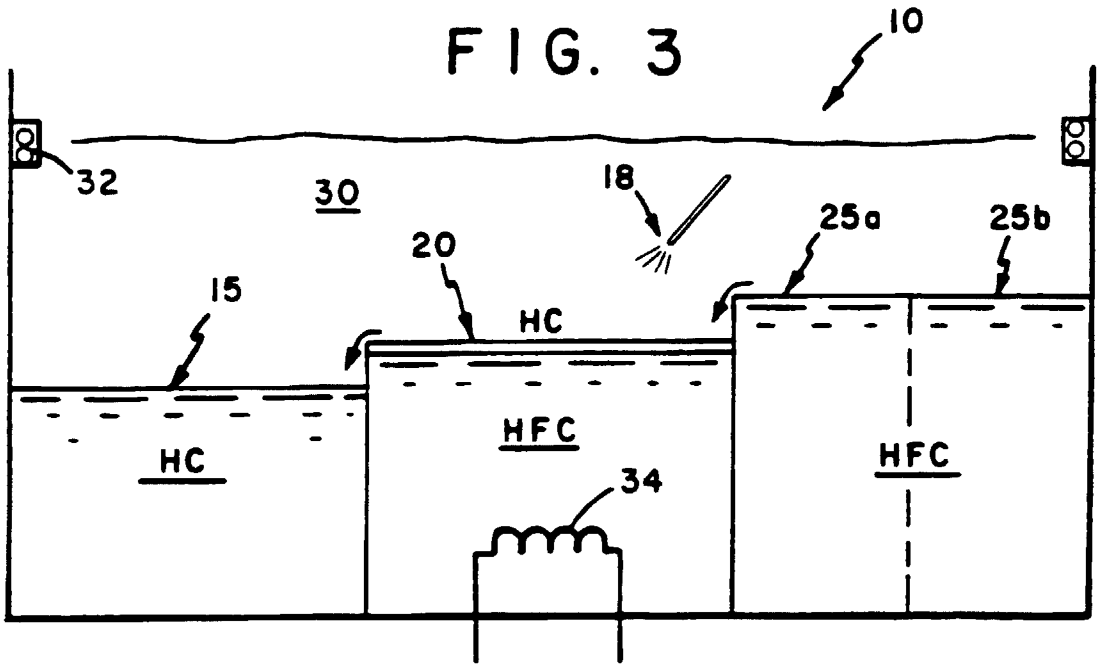
10
32
30
18
20
25a
25b
15
HC
HFC
HC
34
HFC

FIG. 4

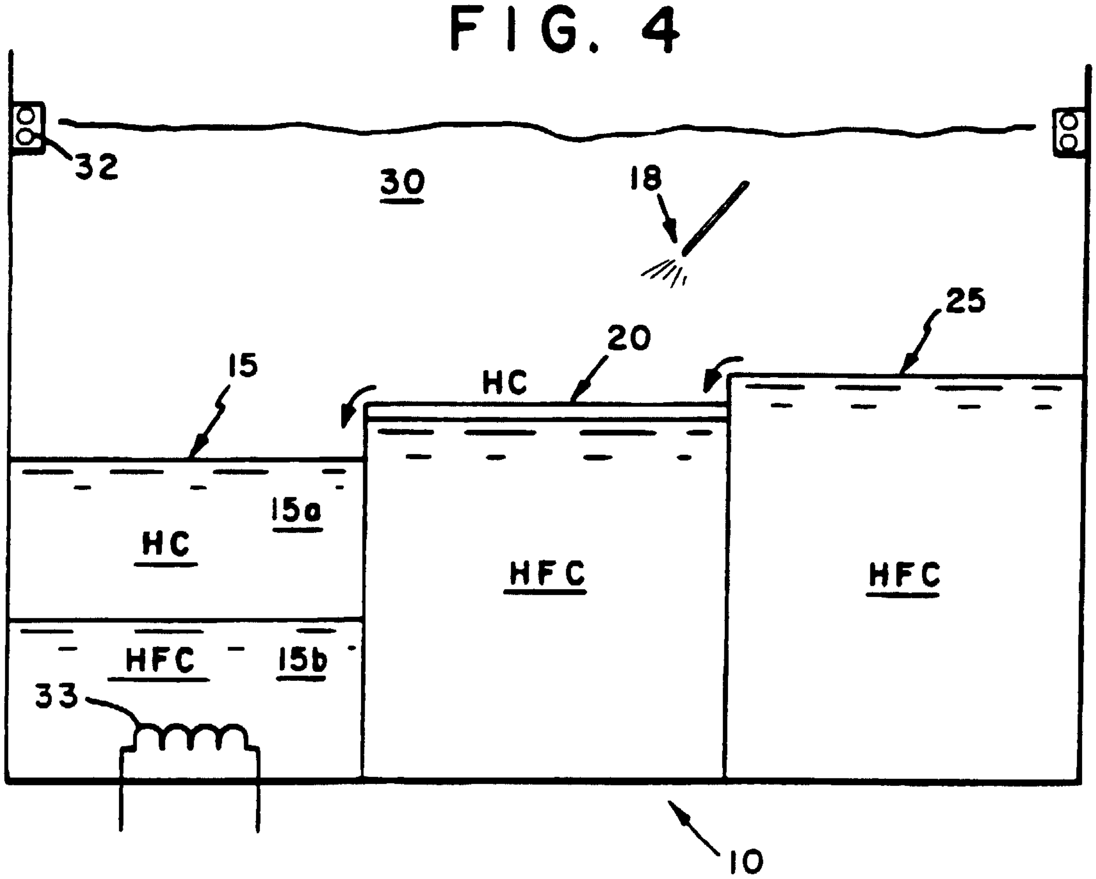
32
30
18
25
20
15
HC
15a
HC
HFC
HFC
HFC
15b
33
10

FIG. 5

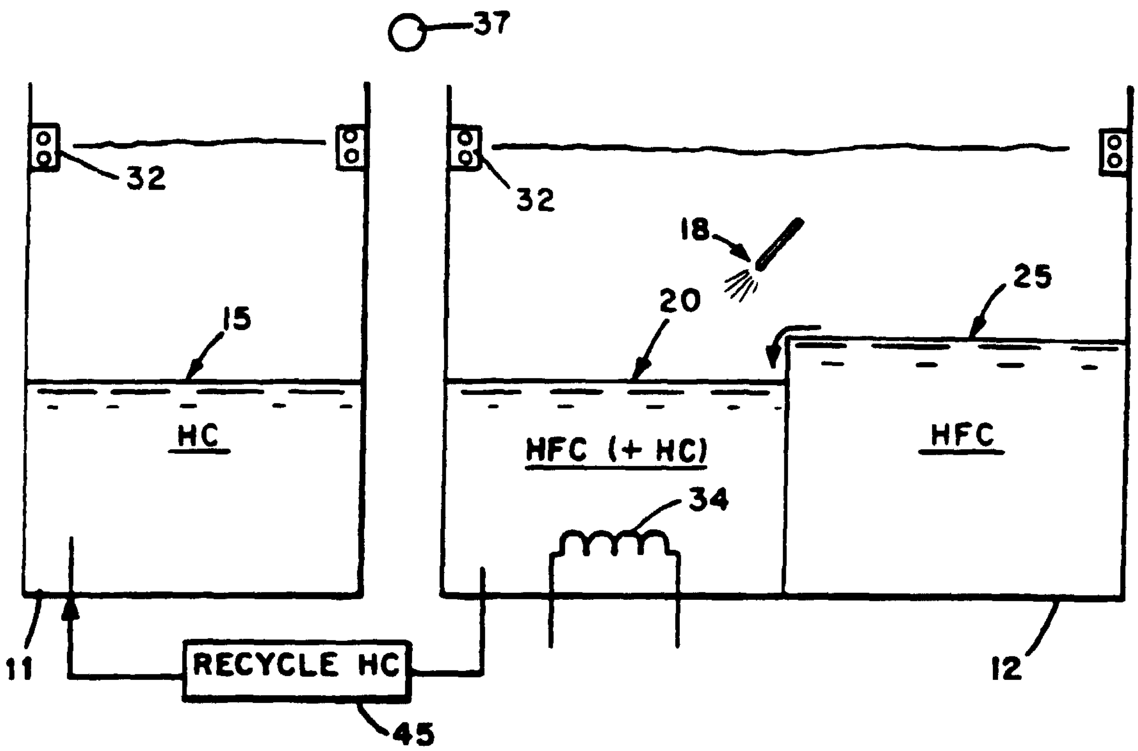
37
32
32
18
25
20
15
HC
HFC (+ HC)
HFC
34
11
RECYCLE HC
12
45

FIG. 6

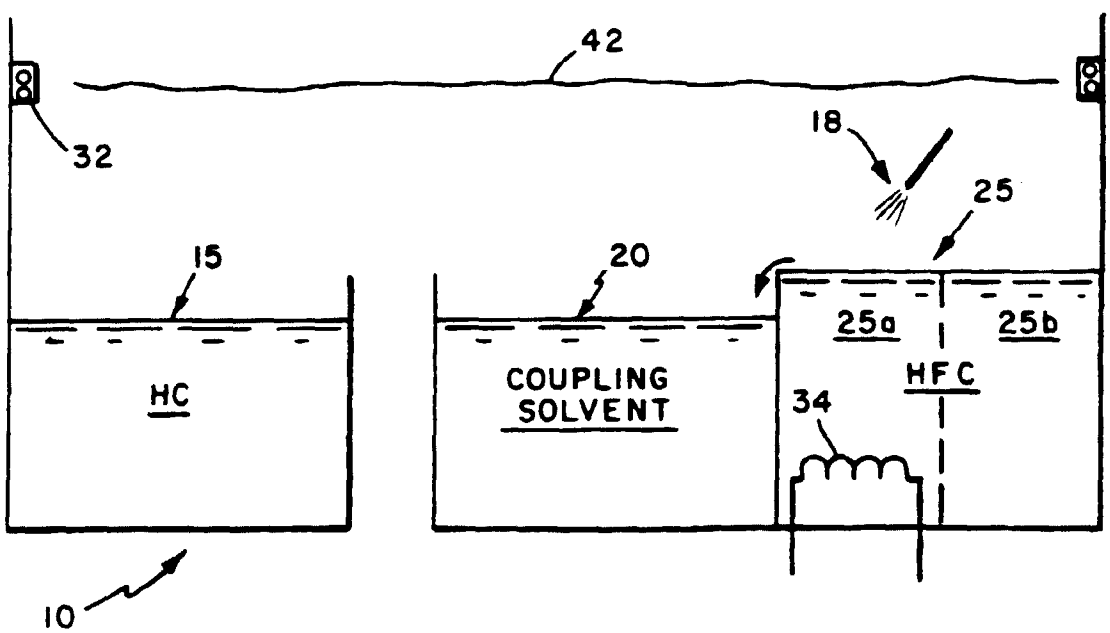
42
32
18
25
15
20
25a
25b
HFC
HC
COUPLING SOLVENT
34
10